(19) **Europäisches Patentamt**
**European Patent Office**
**Office européen des brevets**

(11) **EP 1 894 558 A1**

(12) **EUROPEAN PATENT APPLICATION**
published in accordance with Art. 153(4) EPC

(43) Date of publication:
**05.03.2008 Bulletin 2008/10**

(51) Int Cl.:
***A61K 9/00*** (2006.01)

(21) Application number: **06746884.3**

(22) Date of filing: **26.05.2006**

(86) International application number:
**PCT/JP2006/310555**

(87) International publication number:
**WO 2006/126675 (30.11.2006 Gazette 2006/48)**

(84) Designated Contracting States:
**DE FR GB**

(30) Priority: **27.05.2005 JP 2005155073**
**28.10.2005 JP 2005313896**

(71) Applicant: **KABUSHIKI KAISHA HAYASHIBARA SEIBUTSU KAGAKU KENKYUJO**
**Okayama-shi, Okayama 700-0907 (JP)**

(72) Inventors:
• **Kyono, Fumiyo,**
**KK Hayashibara Seibutsu**
**Okayama-shi,**
**Okayama 7000907 (JP)**

• **Suemoto, Yasuo,**
**KK Hayashibara Seibutsu**
**Okayama-shi,**
**Okayama 7000907 (JP)**

• **Takayama, Satoru,**
**KK Hayashibara Seibutsu**
**Okayama-shi,**
**Okayama 7000907 (JP)**

• **Takeuchi, Makoto,**
**KK Hayashibara Seibutsu**
**Okayama-shi,**
**Okayama 7000907 (JP)**

(74) Representative: **Daniels, Jeffrey Nicholas et al**
**Page White & Farrer**
**Bedford House**
**John Street**
**London, WC1N 2BF (GB)**

(54) **AGENT FOR EXTERNAL APPLICATION TO THE SKIN**

(57) The present invention has an object to provide an agent for suppressing melanin production and/or inhibiting elastase activity containing a plant extract as an effective substance with high safety and acceptable levels of stability and odor or sediment formation and an agent for external application to the skin comprising the above agent which has excellent effects in skin-whitening and skin-beautifying. The object is attained by providing an agent for external application to the skin containing indigo-plant extract.

**EP 1 894 558 A1**

**Description**

## TECHNICAL FIELD

The present invention relates to an indigo-plant extract and an agent for external application to the skin which comprising the indigo-plant extract as an effective ingredient, particularly, an melanin production suppressing and/or elastase activity inhibiting agent comprising indigo-plant extract prepared by extraction with aqueous solvent and an skin-whitening and/or skin-beautifying agent for external application to the skin comprising the above agent as an effective ingredient.

## BACKGROUND ART

Since crease, little wrinkle, fleck, freckle and pigmentation caused by sunburn are major problems of skin for middle-aged persons and women, various skin-beautifying or skin-whitening ingredients have been developed. Followings are well known as the effective substances for skin-beautifying and skin-whitening: chemical substances such as koji acid and its derivatives, L-ascorbic acid, tocopherol and its derivatives, glutathione, hydrogen peroxide, zinc peroxide and hydroquinone monobenzylether, animal extracts such as placental extract and silk extract, and plant extracts such as extract of arnica, althea, aloe, scutellaria, isodonis, chamomile, glycyrrhiza, gardenia, "shikon", bugbane, birch, cindium, geranium, "saiko", sasanqua, Japanese angelica, calendula, sanbucus, safflower, garlic, coix, lycee and logwood.

However, application of these substances in cosmetics does not give satisfactory result because of their low stability or low solubility and, particularly when plant extracts are used, formation of sediment or nasty smell or pigmentation. In respect to effect on living body, satisfactory result has not be obtained because of low safety such as irritating the skin.

Indigo plant is a polygonaceous annual herbaceous plant classified as class *Dicotyledonopsidae* subclass *Archichlamiidae* native to the southern part of Vietnam. Since leaf and fruit of indigo plant have been thought to have physiologically effective substances, indigo-leaf and indigo-fruit, obtained by sun-drying the leaf and fruit of indigo plant, have been used as herbal medicines from ancient times in Japan. In recent years, foods, drinks and cosmetics comprising indigo-plant extract are proposed, which are expected to exert antitumor activity, antiviral activity or antimicrobial activity (for example, q.v. Japanese Patent Kokai No. 31581/2001 and Japanese Patent Kokai No. 189732/2004).

## DISCLOSURE OF INVENTION

An object of the present invention is to provide an agent of suppressing melanin production and/or inhibiting elastase activity comprising plant extract with high safety and acceptable levels of stability, odor and sediment formation, and an agent for external application to the skin comprising the above agents with excellent skin-whitening, skin-beautifying and antiaging effects.

The inventors of the present invention have dedicated to study on plant extract to attain the above object and found out unexpected fact that indigo-plant extract among extracts of polygonaceous plants exhibits effects of suppressing melanin production, inhibiting elastase activity and other physiolosical activities such as inhibiting lipase activity and regulating sebum secretion from glandula sebacea, and that an agent for external application to the skin comprising the above extract exhibits excellent skin-whitening, skin-beautifying and antiaging effects. The present invention was accomplished by finding out unexpected fact that the physiological activities of indigo-plant extract such as antioxidant, anti-inflammatory, antimicorbiol and antiaging effect as well as skin-whitening and beautifying effects are enhanced by its concomitant use with other skin-whitening ingredients.

The present invention attains the above object by providing an agent of suppressing melanin production, inhibiting elastase activity and/or antiaging containing indigo-plant extract and an agent for external application to the skin comprising these agents.

## BRIEF DESCRIPTION OF DRAWINGS

FIG. 1 is a graph showing the relation between concentration of indigo-plant extract and elastase activity inhibiting ratio.
FIG. 2 is a graph showing the relation between concentration of indigo-plant extract and lipase activity inhibiting ratio.
FIG. 3 is a graph showing the relation between concentration of indigo-plant extract and $O^{2-}$ eliminating ratio.
FIG. 4 is a graph showing the relation between concentration of indigo-plant extract and DPPH radical scavenging ratio.

**BEST MODE FOR CARRYING OUT THE INVENTION**

"Indigo plant" used in the present invention is a polygonaceous annual plant Polygonum *tinctorium* Lour, which is called "Chinese indigo".

The indigo plant used in the present invention is not restricted by its origin and cultivation method, and any of naturally growing plant, cultivated plant and a variation created by breeding these by conventional means and a culture obtained by tissue culture, callus culture and cell culture of indigo plant is usable. When a plant body is used as a material for extraction, both whole and parts of the plant body is usable. Any form of the materials such as fresh, i.e. containing water, frozen and dried one and a mixture of these is acceptable.

The indigo-plant extract used in the present invention can be prepared by conventional extraction method used in cosmetic and pharmaceutical industry after adding water or aqueous solvent to whole and/or parts of the above indigo-plant body as material, and obtained as squeezed indigo-plant juice or mixture of the juice and the extract. One or more forms of chipped, crushed, triturated, pulverized, pressed, fermented, dried and frozen indigo-plant can be used as materials for extraction, which are prepared by combinational process of one or more physically and/or chemically pretreatment conventionally used in cosmetic and pharmaceutical industry such as finely-cutting, crushing, trituration, pulverization, pressing, fermentation, drying and freezing before the extraction.

For a solvent for preparing the indigo-plant extract used in the present invention, aqueous solvents (for example, water and acidic or basic aqueous solvents), alcohols (for example, lower alcohols such as methanol, absolute ethanol and ethanol, and polyols such as propylene glycol, 1,3-butylene glycol) and ketones such as acetone can be used by mixing one or more of these solvents. Particularly, water or water-containing solvent (hereinafter, extract of indigo plant by water or water-containing solvent may be collectively referred to as "indigo-plant extract" throughout the specification) are preferable in respect to the levels of skin-whitening and skin-beautifying effect and safety. For the mixed solvents, a solvent consisting of water and ethanol is preferable, its ethanol content is preferable in a range of 0 w/w % to 70 w/w %, more preferably in a range of 0 w/w % to 60 w/w % (hereinafter, "w/w %" is referred to as "%" throughout the specification unless specified otherwise). Adjusting the extracting solvent to adequate pH with acid, base and buffer is acceptable. Extraction is conducted usually in a condition in a range of pH 2 to pH 13, preferably of pH 4 to pH 10, more preferably of pH 4 to pH 7.5.

For the preparation of the indigo-plant extract of the present invention, an adequate amount of the above solvents is used to the materials described above usually in a mass ratio of the solvent to the material of 0.1 to 30, preferably of 0.5 to 20. Then, the indigo-plant extract can be obtained by separating the fluid from the residue by methods such as filtration, centrifugation and decantation, after stirring, heating, pressing or ultrasonication if needed. The extract can be prepared also by repeating the same extraction process on the residue and mixing each obtained fluids. When repeating the extraction on the material or the residue twice or more, the indigo-plant extract is obtainable by collecting desired fluids or mixing fluids after extraction with different solvents in each process. The extraction process by the solvents is usually operated for 5 to 100 hours, preferably for 5 to 48 hours.

When preparing these indigo-plant extracts, one or more agents described later selected from surfactant, preservative (antiseptic), humectant, thickener, water-soluble polymer, antioxidant, chelating agent, pigment, perfume, pH regulator, vitamins including L-ascorbic acid and its derivatives and additives can be added to suppress sediment formation, browning, odor formation and microbial growth. As the timing and amount of adding these ingredients is not restricted as long as effects of the present invention are not suppressed, they can be added at any step in the extracting process.

The indigo-plant extract extracted by the above methods can be used as a material for an agent for external application to the skin without modification. Sediment formation, browning and odor formation can be suppressed by removing the sediments formed during storage for a certain period by methods such as filtration, centrifugation and decantation.

The above extract can be purified to give an indigo-plant extract with elevated content of its effective substances to enhance the efficacy. The extract can be purified to increase or reduce the content of one or more effective substances such as polyphenols, alkaloids, terpenoids, steroids, phenols, pigments, saccharides, proteins, aminoacids, nucleic acids, peptides and lipids according to the intended purpose. The purification methods are selected from the methods such as filtration, concentration, centrifugation, crystallization, solvent fractionation, fractional precipitation, dialysis, hydrophobic chromatography, reversed-phase chromatography, absorption chromatography, affinity chromatography, gel-filtration chromatography and/or ion-exchange chromatography according to the objective ingredients. The indigo-plant extract of the present invention is preferable to contain 200 μg/ml or more, preferably 400 μg/ml or more of polyphenols in respect to the skin-whitening, skin-beautifying and antiaging effects. Also the extract with elevated content of effective substances such as tryptanthrin or kempherol, which have a significant role in skin-whitening, antiaging, antimicrobiol and anti-inflammatory effect, is preferably used.

These indigo-plant extracts can be used in a form such as liquid, solid, powder, paste, gel, semisolid, emulsion and suspension prepared by centrifugation, filtration including ultrafiltration and hyper filtration, concentration and drying if needed or by adding pharmaceutically applicable ingredients. For concentration and drying, conventional means used in food, cosmetic, and pharmaceutical industry such as vacuum concentration, fine microfiltration membrane concentration, reverse osmosis membrane concentration, ultrafiltration concentration, vacuum drying, lyophilization and spray drying are usable in combination. When the indigo-plant extract is dried, the extract mentioned above can be dried without modification, however cyclodextrin, cyclic tetrasaccharide, cyclic pentasaccharide, $\alpha,\alpha$-trehalose and its derivatives, and saccharide comprising these saccharides can be preferably used as vehicle or stabilizer, more preferably cyclodextrin can be used since homogeneous powder can be prepared with small amount of cyclodextrin.

The indigo-plant extract prepared by the above methods have at least the following effects as well as effects in suppressing melanin production, inhibiting elastase activity, antiaging, inhibiting lipase activity and regulating sebum secretion from glandula sebacea:

(1) scavenging intravital radicals derived from active oxygen and lipid peroxide, which may cause diseases such as malignant tumor, mycocardial infraction, cerebral stroke, rheumatism, lifestyle-related disease (adult disease), renal defect and hemorrhoid, stress and aging;

(2) regulating cytokine production by immunocompetent cell, which is involved in a intravital balance of helper T-cell type 1 (Th1) and helper T-cell type 2 (Th2) including interferon-$\gamma$ and interleukin-10, to bring the balance into the normal condition;

and then therapying and preventing diseases such as autoimmune disease, hepatic defect, renal defect, pancreatic defect, graft-versus-host disease caused by the abnormal balancce;

(3) inhibiting the expression of activities of carbon monoxide forming enzyme and prostaglandin forming enzyme in intravital cells induced by stimulus of ultraviolet, cytokine and endotoxin to suppress the formation of carbon monoxide and prostaglandin $E_2$;

and then exerting the skin-whitening effect by suppression of melanin production in melanocyte caused by stimulus of the carbon monoxide and prostaglandin and also therapying and preventing diseases such as autoimmune disease, allergosis, inflammatory disease, malignant tumor, renal defect and pulmonary defect involved in by excess carbon monoxide and prostaglandin in the living body.

The indigo-plant extract above mentioned can regulate vital functions to suppress the inflammation, which is accompanied by disorders of biomedical tissue caused by infection of microbial such as gram-positive bacteria, gram-negative bacteria, fungi and virus, invasion or contact of xenobiotic matter such as protein, organic compound and metal and tumor formation by suppressing the production of inflammatory cytokines including tumor necrosis factor (TNF), interferon-$\gamma$ and interleukine-1. The effective substances such as tryptanthrin contained in the indigo-plant extract above mentioned have strong bacteriostasis and/or bactericidal activity against periodontal disease-causing bacteria such as *Prophyromonas gingivalis* (hereinafter, may be abbreviated as "P. *gingivalis")* and cariogenic bacteria such as Streptococcus *mutans* (hereinafter, may be abbreviated as "*S. mutans"),* which exist on tooth surface and periodontal pocket. Therefore, when used for oral external agent, it can prevent and relieve periodontal disease and caries by inhibiting swelling and inflammation of gum-ridge and oral bleeding and improving oral plaque control, and also can reduce the risk of developing of pneumonia caused by aspiration or oral bacteria, kidney inflammation, septicemia, bacteremia, bacterial carditis, arterial diseases, birth of low-weight baby, diabetes and esophageal cancer. In addition to as an agent of suppressing melanin production, inhibiting elastaze activity, antiaging, inhibiting lipase activity and regulating sebum secretion from glandula sebacea, the indigo-plant extract of the present invention can exert mildly anti-microbial effect on microbes causing athlete's foot and acne, anti-inflammatory effect for skin disease such as stomatitis, dermatitis, eczema and rash, antiaging effect, periodontal disease suppressing effect, cariostatic effect, active oxygen eliminating effect, radical scavenging effect, inhibitory or regulatory effect on cytokine production, inhibitory effect on carbon monoxide forming enzyme expression, inhibitory effect on prostaglandin forming enzyme activity and/or regulatory effect on vital functionsbe, and can be used as an cosmetic, medical or medicated-cosmetic agent for external application to the skin for healthy or sick persons, which, and also can be used as an material for production of food and drinks, feed, bait, pet food and general merchandise. Since the indigo-plant extract of the present invention exhibits beneficial effect in accelerating hair papilla cell growth, it can be used as an effective substance for hair-growth drug, and also the graft survival of inplanted hair root on scalp can be increased when the hair root is transplanted to scalp after immersing the hair root resected from scalp in a liquid containing the indigo-plant extract.

The content of the indigo-plant extract of the present invention in the agent for external application to the skin is not restricted as long as the desired effect is exhibited, and can be determined according to the efficacy or agent forms. The content of the extract is usually preferable to be 0.0005% to 30%, preferably 0.005% to 10%, morepreferably 0.05% to 10% of the total amount of the agent for external application to the skin, on a dry solid basis. The desired effect can not be obtained when the content is less than 0.0005%, and the effect does not increase any more and physical property of some composition forms may be affected when the content is 30% or higher.

When the indigo-plant extract of the present invention is used for an agent for external application to the skin in combination with one or more other skin-whitening ingredients, the enhanced effects in antioxidation, anti-inflammation and antimicroorganism, which are the own properties of indigo-plant extract, as well as synergistically enhanced effect in inhibiting melanin production, inhibiting elastase activity, inhibiting lipase activity and/or antiaging. For skin-whitening ingredients except the indigo-plant extract, any ingredient is usable with no restriction of origin and preparation method as long as it has skin-whitening effect and enhances the physiological activity of the indigo-plant extract, for concrete example, L-ascorbic acid, its derivatives and their salts, alkoxysalicylic acid and its salts, hydroquinone glycosides and its derivatives, tranexamic acid, its derivatives and their salts, resorcinol derivatives, koji acid, its derivatives and their salts, ellagic acid and its salts, linoleic acid and its salts, camomile extract and tetrahydrocurcuminoid are quoted. In respect to the level of enhancing effect on the physiological activities of the indigo-plant extract, ascrobic acid, its derivatives and their salts are preferably used, and L-ascorbic acid 2-glucoside is more preferably used.

When one or more of the above skin-whitening ingredients are concomitantly used together with the indigo-plant extract, their content in the agent for external application to the skin is not restricted as long as no pharmaceutical problem occurs. They are usually used in an amount of 0.001% to 20% of the total amount of the agent for external application to the skin. The enhancing effect on skin-whitening effect of the agent for external application to the skin tends to decrease when the content is lower than 0.001%, and enhancement of the effect may not increase anymore and admixing of them in the agent become difficult even when the content is higher than 20%. They can be used more preferably in a content of 0.01% to 10%, most preferably of 0.1% to 10%. When camomile extract is used, the above value is obtained on the dry solid basis.

Any form of L-ascorbic acid and its derivatives is usable in the present invention. For concrete examples, L-ascorbic acid, its derivatives such as L-ascorbic acid alkylesters including L-ascorbic acid monostearate, L-ascorbic acid monopalmitate, L-ascorbic acid monooleate, L-ascorbic acid distearate, L-ascorbic acid dipalmitate and L-ascorbic acid dioleate, L-ascorbic acid phosphate and its salts including L-ascorbic acid phosphate magnesium salt and L-ascorbic acid phosphate ester sodium salt, L-ascorbic acid sulfate and its salts, and L-ascorbic acid 2-glucoside and its acyl derivatives, among which L-ascorbic acid 2-glucoside is preferable, are quoted. These compounds can be used in salt forms such as ammonium salt and amino acid salt as well as alkali metal salts and alkali earth metal salts such as sodium salts, potassium salts, magnesium salts and calcium salts. Among the salts of L-ascorbic acid phosphate, alkaline earth metal salts are preferable, and magnesium salt is more preferable. For the L-ascorbic acid derivatives used in the present invention, one or more compounds selected from L-ascorbic acid alkylesters, L-ascorbic acid phosphate and its salts, L-ascorbic acid sulfate and its salts and L-ascorbic acid 2-glucoside and its salts can be preferably used.

Alkoxysalicylic acids used in the present invention are salicylic acids in which any hydrogen atoms at C-3, C-4 or C-5 position are substituted by alkoxyl groups. Among the above alkoxyl groups, methoxyl group, ethoxyl group, propoxyl group, isopropoxyl group, butoxyl group and isobutoxyl group are preferable. Particularly, methoxyl group and ethoxyl group are more preferable.

For concrete examples of alkoxysalicylic acids, 3-methoxysalicylic acid (2-hydroxy-3-methoxybenzoic acid), 3-ethoxysalicylic acid (2-hydroxy-3-ethoxybenzoic acid), 4-methoxysalicylic acid (2-hydroxy-4-methoxybenzoic acid), 4-ethoxysalicylic acid (2-hydroxy-4-ethoxybenzoic acid), 4-propoxysalicylic acid (2-hydroxy-4-propoxybenzoic acid), 4-isopropoxysalicylic acid (2-hydroxy-4-isopropoxybenzoic acid), 4-butoxysalicylic acid (2-hydroxy-4-butoxybenzoic acid), 5-methoxysalicylic acid (2-hydroxy-5-methoxybenzoic acid), 5-ethoxysalicylic acid (2-hydroxy-5-ethoxybenzoic acid) and 5-propoxysalicylic acid (2-hydroxy-5-propvxybenzoic acid) are quoted. In respect to the level of skin-whitening effect, 4-methoxysalicylic acid is particularly preferable.

For the preparation of the agent for external aplication to the skin of the present invention, alkoxysalicylic acids can be used as a form of the salt. The salts of 4-methoxysalicylic acid are preferable. The salts are not restricted, for example, alkali metal or alkali earth metal salts such as sodium salt, potassium salt, calcium salt, ammonium salt and amino acid salts are quoted. For the present invention, potassium salt is preferable, and potassium 4-methoxysalicylate is more preferable.

For the hydroquinone glycosides used in the present invention, glycosides of 6-carbon sugars such as hydroquinone-$\alpha$-D-glucoside, hydroquinone-$\beta$-D-glucoside, hydroquinone-$\alpha$-L-glucoside, hydroquinone-$\beta$-L-glucoside, hydroquinone-$\alpha$-D-galactoside, hydroquinone-$\beta$-D-galactoside, hydroquinone-$\alpha$-L-galactoside and hydroquinone-$\beta$-L-galactoside, glycosides of 5-carbon sugars such as hydroquinone-$\alpha$-L-riboside, hydroquinone-$\beta$-L-riboside, hydroquinone-$\alpha$-D-arabinoside, hydroquinone-$\beta$-D-arabinoside, hydroquinone-$\alpha$-L-arabinoside and hydroquinone-$\beta$-L-arabinoside, glycosides of amino sugars such as hydroquinone-$\alpha$-D-glucosaminide, hydroquinone-$\beta$-D-glucosaminide, hydroquinone-$\alpha$-L-glucosaminide, hydroquinone-$\beta$-L-glucosaminide, hydroquinone-$\alpha$-D-galactosaminide hydroquinone-$\beta$-D-gatactosaminide, hydroquinone-$\alpha$-L-galactosaminide and hydroquinone-$\beta$-L-galactosaminide, and glycosides of uronic acids such as hydroquinone-$\alpha$-D-glucuronide, hydroquinone-$\beta$-D-glucuronide, hydroquinone-$\alpha$-L-glucuronide, hydroquinone-$\beta$-L-glucuronide, hydroquinone-$\alpha$-D-galacturonide, hydroquinone-$\beta$-D-galacturonide, hydroquinone-$\alpha$-L-galacturonide-andhydroquinone-$\beta$-L-galacturonideare quoted.

For the hydroquinone glycosides used in the present invention, hydroquinone-$\beta$-D-glucoside and/or hydroquinone-$\alpha$-D-glucoside are preferably used in respect to the level of skin-whitening effect, availability and safety, hydroquinone-β-D-glucoside (hereinafter, referred to as its general name "arbutin") is more preferable.

For the derivatives of the hydroquinone glycosides, esters such as aceylated compounds and ethers such as methylated compounds are quoted.

For the tranexamic acid derivatives used in the present invention, for example, dimer of tranexamic acid (such as including trans-4-(trans-aminomethylcyclohexanecarbonyl)aminomethylcyclohexa necarboxylic acid hydrochloride), esters of tranexamic acid and hydroquinone (such as trans-4-aminomethylcyclohexanecarboxylic acid 4'-hydroxyphenyl ester), esters of tranexamic acid and gentisic acid (such as 2-(trans-4-aminomethycyclohexylcarbonyloxy)-5-hydroxybenzoic acid and its salts), amides of tranexamic acid (such as trans-4-aminomethylcyclohexanecarboxylic acid methylamide and its salts, trans-4-(*p*-methoxybenzoyl)aminomethylcyclohexanecarboxylic acid and its salts, and trans-4-guanidinomethylcyclohexanecarbosylic acid and its salts) are quoted.

For the resorcinol derivatives used in the present invention, for major example, alkylresorcinols are quoted, for concrete example, 4-alkylresorcinols are preferable, and 4-n-butylresorcinol is more preferable.

The origin and preparation method of koj i acid and its derivatives used in the present invention are not restricted. For the koji acid derivatives, for example, koji acid esters such as koji acid alkylesters, koji acid ethers such as koji acid alkylethers and koji acid glycosides are quoted. For concrete example of koji acid ethers, 2-methoxymethyl-5-hydroxy-4H-pyran-4-one, 2-ethoxymethyl-5-hydrosy-4H-pyran-4-one, 2-benzoyloxymethyl-5-hydrosy-4H-pyran-4-one, 2-cinnamoyloxymethyl-5-hydrosy-4H-pyran-4-one and 2-phenoxymethyl-5-hydrosy-4H-pyran-4-one are quoted. For concrete example of the koji acid esters, koji acidpalmitate and koji acid stearate are quoted. For the koji acid glycosides, koji acid compounds in which a saccharide binds to -CH$_2$OH group at C-2 position of koji acid are quoted. For the saccarides, 6-carbon sugars such as glucose, galactose, mannose, fructose and sorbose, 5-carbon sugars such as ribose, arabinose, xylose, lyxose and xylulose, amino sugars such as glucosamine, mannnosamine and galactosamine, disaccharides such as maltose, lactose, cellobiose and sucrose, and trisaccharides such as maltotriose and cellotriose are quoted. Among these, koji acid is preferably used, which has strong enhancing effect on skin-whitening.

The camomile extract used in the present invention is prepared by extraction with a suitable solvent. It can be used after treated by a process such as concentration, deodorization, purification or drying as long as the effect of the present invention is not lost. Also commercially available camomile extract is usable, for example, "Camomile liquid" (produced by Ichimaru Pharcos Co., Ltd., solid content is 0.6% to 1.2%).

For the tetrahydrocurcuminoids used in the present invention, for example, compounds described in Japanese Patent Kokai No. 128133/1994 and Europe Patent Application No. 1108419 are quoted. For concrete example, tetrahydrocurcumin, tetrahydrocurcumin, tetrahydrodemetoxycurcumin, tetrahydrocurcumin, and tetrahydro-bis-demetoxycurcumin are quoted.

Among the tetrahydrocurcuminoids used in the present invention, tetrahydrocurcumin is preferable, which can be obtained as a commercial product, for example, "Tetrahydrocurcuminoid" (produced by SABINSA Corporation).

The indigo-plant extract and its mixture with other skin-whitening ingredients mentioned above, which are obtained by the present invention, can be used as an agent for external application to the skin directly, and can be also admixed with one or more of other pharmaceutically or physiologically applicable ingredients as long as the effect of the present invention is not inhibited. For these ingredients, for example, water, alcohols, oils, surfactants, preservatives (antiseptics), perfumes, humectants, thickeners, water-soluble polymers, antioxidants, chelating agents, pH regulators, foaming agents, ultraviolet absorpting and scattering agents, fine particles, vitamins, amino acids, anti-inflammatory agents, seaweed extracts, additives for medicine, medicated cosmetics, cosmetics and food, and effective substances for medicine and medicated cosmetics are quoted.

For concrete example of oils, botanical oils such as macadamia nut oil, castor oil, olive oil, cacao oil, camellia oil, palm oil, sumac wax, jojoba oil, grape seed oil and avocado oil, animal oils such as mink oil and egg yolk oil, waxes such as beeswax, whale wax, lanoline, carnauba wax and candelilla wax, hydrocarbons such as liquid paraffin, squalene, microcrystalline wax, ceresin wax, paraffin wax and vaseline, natural and synthetic fatty acids such as capric acid, myristic acid, palmitic acid, stearic acid, behenic acid, lanolic acid, linoleic acid, linolenic acid, lauric acid, myristic acid, oleic acid and isostearic acid, natural and synthetic higher alcohols such as cetanol, stearyl alcohol, hexyldecanol, octyldodecanol, lauryl alcohol, capryl alcohol, myristyl alcohol, cetyl alcohol, cholesterol and phytosterol, and esters such as myristic acid isopropyl, palmitic acid isopropyl, myristic acid octyldodecyl, oleic acid octyldodecyl and choresterol oleate are quoted.

For the surfactants, nonionic surfactants such as monolauric acid sorbitan, monopalmitic acid sorbitan, sesquioleic acid sorbitan, trioleic acid sorbitan, monolauric acid polyoxyethylenesorbitan, monostearic acid polyoxyethylenesorbitan, polyethyleneglycol monooleate, polyehtyleneglycol alkylate, polyoxyethylene alkyl ether, polyglycol diether, lauroyl diethanolamide, fatty acid isopropanolamide, maltitol hydroxyfatty acid ether, alkylated polysaccharide, alkylglucoside and sugarester, nonionic surfactants such as lipophilic glycerol monostearate, self-emulsifying glycerol monostearate, polyglycerol monosteatate, polyglycerol alkylate, sorbitan monooleate, polyethyleneglycol monostearate, polyoxyethylenesorbitan monooleate, polyoxyethylenated cetylether, polyoxyethylenated sterol, polyoxyethylenated lanoline, polyoxyethylenated beeswax and polyoxyethylene hydrogenated castor oil, anionic surfactants such as sodium stearate, potassium palmitate, sodium cetylsuflate, sodium laurylphosphate, triethanolamine palmitate, sodium polyoxyethylenelaurylphosphate, sodium N-acylglutamate, sodium palmitate, sodium laurate, sodium laurylate, potassium laurylate, alkylsulfate triethanolamine ether, Turkey red oil, lineardodecylbenzene sulfate, polyoxyethylene hydrogenated castor oil maleate and acylmethyltaurine, cationic surfactants such as stearyldimethylbenzylammonium chloride, stearyltrimethylammonium chloride, stearyltrimethylammonium chloride, benzalkonium chloride and laurylamine oxide, and ampholytic surfactants such as alkylaminoethylglycine-hydroohloride solution and lecithin are quoted.

For the preservatives (antiseptics) , benzoic acid and its salts, salicylic acid and its salts, sorbic acid and its salts, dehydroacetic acid and its salts, p-oxybenzoic acid ester such as p-oxybenzoic acid alkylester, 2,4,4'-trichloro-2'-hydroxydiphenylether, 3,4,4'-trichlorocarbanilide, hexachlorophenone, benzalkonium chloride, phenoxyethanol, hinokitiol, resorcinol, ethanol, 1,3-butyleneglycol and photosensitizer 201 are quoted. Particularly, 1,3-butyleneglycol can be used for effectively suppressing production of sediment and nasty smell as well as for suppressing microbial growth, when added at a concentration of 0.1% to 40%, more preferably of 1% to 30% of the indigo-plant extract.

For the perfumes, benzaldehyde, benzylbenzoate, phenylacetic acid, sandalol, eugenol, lilial, linalool, 2-methyl-3-(4-methylphenyl)-propanal, musk ketone, cinnamic aldehyde, vertofix, methylionone, geranyl formate, Iso E Super, $\gamma$-undecalactone, hexylsalicylate, cis-3-hexenylsalicylate, methyldihydrojasmonate, tetrahydrofurfryl-3-mercaptopropionate, kovanol, vanillin, vanillal, geranium oil, pennyroyal oil, birch oil and armoise oil are quoted. These perfumes can be advantageously used for improving odor of the agent for external application to the skin of the present invention by masking of the characteristic odors of the plant extracts including indigo-plant extract.

For humectants, polyols such as glycerol, erythritol, xylitol, maltitolglycerol, propyleneglycol, 1,3-butyleneglycol, sorbitol, maltitol, polyglycerol, polyethyleneglycol, dipropyleneglycol, 1,2-pentanediol and isopropyleneglycol, saccharides such as glucose, maltose, trehalose, sugar derivatives of trehalose, dextrin, cyclodextrin, branched cyclodextrin, cyclic sugars including cyclotetrasaccharide having structure of cyclo{-6)-$\alpha$-D-glucopyranosyl-(1-3)-$\alpha$-D-glucopyranosyl-(1-6)-$\alpha$-D-glucopyranosyl-(1$\rightarrow$3)-$\alpha$-D-glucopyranosyl-(1$\rightarrow$} (cyclonigerosylnigerose), which is disclosed in the specification of WO02/10361, cyclotetrasaccharide having structure of cyclo($\rightarrow$6)-$\alpha$-D-glucopyranosyl-(1$\rightarrow$4)-$\alpha$-D-glucopyranosyl-(1$\rightarrow$6)-$\alpha$-D-glucopyranosyl-(1$\rightarrow$4)-$\alpha$-D-gludopyranosyl-(1$\rightarrow$) (cyclomaltosylmaltose), which is described in Japanese Patent Kokai No. 95148/2005 and cyclicpentasaccharide and cyclichexasaccharide having structure of cyclo{$\rightarrow$6)-[$\alpha$-D-glucopyranosyl-(1 $\rightarrow$4)]$_n$-$\alpha$-D-glucopyranosyl-(1$\rightarrow$} (n means 4 or 5), which is disclosed in the specification of PCT/JP2005/17642 (q.v. WO2006/035725A1), natural moisturizing factors (NMF) such as amino acid, peptide, sodium lactate and sodium pyrrolidonecarboxylate, water-soluble polymers such as xyloglucan, quince seed, carrageen, pectin, mannan, curdlan, galactan, dermatan sulfate, glycogen, keratan sulfate, chondroitin, chondroitin sulfate, mucoitin sulfate, kerato sulfate, locust bean gum, succinoglucan, charoninic acid, hyaluronic acid, heparan sulfate, sodium hyaluronate, collagen and mucopolysaccharide, silicones such as dimethylpolysiloxane and methylphenylsiloxan, culture supernatants of lactic acid bacteria and bifidbacteria are quoted. Particularly, one or more of trehalose, sacchatride derivatives of trehalose, cyclic tetrasaccharide, hyaluronic acid and its salts and chondroitin acid and its salts, which have large moisturizing effect, can be preferably used in combination.

For the thickeners, natural polymers such as sodium alginate, xanthan gum, aluminum silicate, quince seed extract, gum arabic, hydroxyethyl guar gum, carboxymethyl guar gum, guar gum, dextran, tragacanth gum, cellulose, starch, pullulan, chitin, chitosan, carboxymethylchitin and agar, semi-synthetic polymers such as hydroxypropylcellulose, methylhydroxypropylcellulose, methylcellulose, carboxymethylcellulose, hydroxyethylcellulose, soluble starch, branched starch and cationized cellulose, synthetic polymers such as carboxyvinylpolymer, polyvinylalcohol, polyvinylpyrrolidone and vinylalcohol-vinylacetate copolymer are quoted.

For the antioxidants, dibutylhydroxytoluene, butylhydroxyanisole, propylgallate, L-ascorbic acid, vitamin E, vitamin P, catechins, flavonoids and these derivatives are quoted.

For the chelating agents, disodium edetate, ethylenediamintetraacetate, pyrophosphate, hexametaphosphate, citric acid, tartaric acid and gluconic acid are quoted.

For pH regulator, sodium hydroxide, potassium hydroxide, triethanolamine, nitrilotriethanol, citric acid, sodium citrate, boric acid, borax and potassium hydrogen phosphate are quoted.

For the ultraviolet absorbing and scattering agents, ultraviolet absorber related to p-aminobenzoic acid, ultraviolet absorber related to anthranilic acid, ultraviolet absorber related to salicylic acid, ultraviolet absorber related to cinnamic acid, ultraviolet absorber related to benzophenone, ultraviolet absorber related to saccharide, 3-(4'-methylbenzylidene)-d-camphor, 3-benzylidene-d,1-camphor, urocanic acid, urocanic acid ethylester, 2-phenyl-5-methylbenzoxazole, 2,2'-hydroxy-5-methylphenylbenzotriazole, 2-(2'-hydroxy-5'-t-octylphenyl)benzotriazole, 2-(2'-hydroxy-5'-methylphenyl)benzotriazole, dibenzaladine, dianisoylmethane, 4-methoxy-4'-t-butyldibenzoylmethane, 5-(3,3-dimethyl-2-norbornylidene)-3-pentan-2-one, 2-hydroxy-4-methoxybenzophenone, octyldimethyl-p-aminobenzoate, ethylhexyl-p-methoxycinnamate, titanium oxide, kaolin and talc are quoted.

For vitamins except L-ascorbic acid and its derivatives, vitamin A and its derivatives, vitamin B family such as vitamin $B_1$ and its derivatives, vitamin $B_2$ and its derivatives, vitamin $B_6$ hydrochloride, vitamin $B_6$ tripalmitate, vitamin $B_6$ dioctanoate, vitamin $B_{12}$, and vitamin $B_{15}$ and its derivatives, vitamin E group such as $\alpha$-tocopherol, $\beta$-tocopherol, $\gamma$-

tocopherol and vitamin E acetate, vitamin D group, vitamin H, pantothenic acid, pantethine, vitamin F, vitamin K, bioflavonoids and its derivatives such as rutin, hesperidin and nalingin, vitamin U, ferulic acid, $\gamma$-oryzanol, $\alpha$-lipoic acid, orotic acid, coenzyme Q10, their derivatives and their salts are quoted.

For amino acids, glycine, alanine, valine, leucin, isoleucine, serine, threonine, phenylalanine, tyrosine, asparagine, glutamine, taurine, tryptophan, cystine, cysteine, methionine, proline, hydroxyproline, asparatic acid, glutamic acid, arginine, histidine, lysine, carnitine and their derivatives and their salts are quoted.

For the anti-inflammatory agents, allanthoin and its derivatives such as allanthoin, allanthoinacetyl-dl-methionine, allanthoin chlorohydroxyaluminium, allanthoin dihydroxy aluminium and allantoin polygltacuronic acid, glycyrrhetin and its derivatives such as glycyrrhetinic acid, glycyrrhizinic acid, allanthoin glycyrrhetinate, glycerol glycyrrhetinate, stearyl glycyrrhetinate, glycyrrhetinyl stearate, disodium 3-succinyloxyglycyrrhetinate, dipotassium glycyrrhizinic acid, monoammonium glycyrrizinic acid, derivatives of pantothenic acid such as pantothenic acid, pantothenyl alcohol, pantothenyl ethylether, acetylpantothenyl ethylether, benzoylpantothenyl ethylether, calcium pantothenate, acetylpantothenyl ethylether, benzoate pantothenyl ethylether ester and pantethine, vitamin E derivatives such as vitamin E, d-$\delta$-tocopherol, dl-$\alpha$-tocopherol, dl-$\alpha$-tocopherol acetate, dl-$\alpha$-tocopherol linolate, dl-$\alpha$-tocopherol nicotinate and dl-$\alpha$-tocopherol succinate, L-ascorbic acid derivatives and its salts of alkali metals and alkali earth metals such as L-ascorbicacid, L-ascorbic acid-glycoside including L-ascorbic acid-2-glucoside, acyl derivatives of L-ascorbic acid-glycoside, tetrehaxyldecanoic L-ascorbate, L-ascorbic acid-tocopherol phosphate diester, in which L-ascorbic acid bound to tocopherol by the intermediary of phosphate group, L-ascorbic acid sulfate ester, ascorbyl dipalmitate, ascorbyl palmitate, stearyl L-ascorbate, L-ascrobyl phosphate, ethyl L-ascorbate and their acyl derivatives, pyridoxine hydrochloride, menthol, biotin, camphor, turtenpine, zinc oxide, azulene, guaicazulene and its derivatives, mefenamic acid and its deirvatives, phenylbutazone and its derivatives, indomethacin and its derivatives, ibuprofen and its derivatives, ketoprofen and its derivatives, $\varepsilon$-aminocaproic acid, sodium diclofenac, diphenhydramine, tranexamic acid and its derivatives, dexamethasone, cortisone and its esters, hydrocortisone and its esters, adrenocortical hormons such as prednisone and prednisolone, antihistamic agent, plants and plant-derived substances such as rose fruit, "ibutoranoo", turmeric, hypericum, corktreebark, licorice, honeysuckle, cress, comfrey, acanthopanax, sage, "shikon", birch, tea, calendula, sanbucus, cattail, soapberry, blue gum extract, calabrese, Japanese angelica, Japanese medlar leaf, perilla, camomile, mugwort, aloe, gensing, pellodendron bark powder, myrica powder, cube gambir, sweet tea, althea, arnica, echinacea, isodonis, scutellaria, barley, Sant john's wort, orange, valerian, roman camomile, "kawarayomogi", cucumber, gardenia, "kumazasa", gentian, geranium, burdock, comfrey, chinese pepper, perilla, lime tree, peony, ivy, juniper, western milfoil, pepermint, cindium, sialid, sage, mulberry, jujube, thyme, beningasa seed, calendula, peach kernel, "dokudami", tormentil, gensing, parsley, mint, nettle, sandalwood, Japanese medlar, buche's broom, grape, safflower, peony, lime tree, horse chesnut, peach, cornflower, mugwort, lavender, rosemary, Japanese medlar, carrot and Japanese angelica are quoted.

For the seaweed extracts, extracts of brown algae, red algae, green algae and blue-green algae, for concrete example, laminaria, kelp, brown seaweed, "hijiki", "tengusa", nullipore, palmaria, carageenmoss, laver, ulva, "anaaosa", ascophyllum, fucoid, "mozuku", "okinawamozuku" and himanthalia are quoted, which include extract from aquatic plants such as eel grass.

The agent for external application to the skin of the present invention can be added with one or more agents such as blood circulation improver, astringentia, antiwrinkle agent, cellular stimulant, antiaging agent, hair growth agent, hair regrowth agent and transdernal absorption improver in combination in addition to the ingredients described above. For concrete example, plant or animal constituents except the indigo-plant extract such as propolis, herbs including Chinese parsley and royal jelly and their extract, deep seawater, minerals such as brine and its components, inorganic powder such as antibiotic, calcium hydrogen phosphate, organic modified montmorillonite, silicic acid, anhydrous silicic acid, magnesium silicate, mica, bentonite, mica coated by titanium, bismuth oxychloride, zirconium oxide, magnesium oxide, zinc oxide, calcium carbonate, magnesium carbonate, iron oxide, ultramarine blue pigment, iron blue pigment, chrome oxide, chrome hydroxide, calamine, zeolite and carbon black, polyamide, polyester, polyethylene, polypropylene, polystyrene, polyurethane, vinyl resin, ureaformaldehyde resin, phenol resin, fluorine resin, silicon resin, acrylic acid resin, melamine resin, epoxide resin, polycarbonate resin, divinylbenzen-styrene copolymer, copolymer consist of two or more of the monomer of the above compounds, protein and scleroprotein including silk, organicpowder such as cellulose, natural dyes such as anthraquinone, anthocyanin, chalkone and charotenoid pigments including safflower pigment, gardenia pigment, "shikon" pigment, cochineal, photosensitizers such as photosensitizer 101, 201, 301 and 401, organic pigment powders such as red No. 201, 202, 204, 205, 220, 226, 228 and 405, orange No. 203 and 204, yellow No. 204 and 401 and blue No. 404, organic pigment powders such as zirconium, barium and aluminum rake of red No. 3, 104, 106, 227, 230, 401 and 505, orange No. 205, yellow No. 4, 5, 202 and 203, green No. 3 and blue No. 1, powder of the above organic substances supported by dyes such as natural pigment including "shikon" pigment and safflower pigment, and organic synthetic pigment, and functional ingredients such as rutin glycoside, hesperidin glycoside, gricyrrhizin glycoside, narindin glycoside, esculetin, esculin and esculin glycoside are quoted.

The additive amount of these substances to the agent for external application to the s kin can be determined according to the purpose without restriction as long as the property and/or efficacy of the agent are not affected. Usually, they can be used in a concentration of 0.0001% to 99%, preferably of 0.001% to 70%, more preferably of 0.01% to 30% of the agent for external application to the skin.

Any form of the agent for external application to the skin is acceptable, for example, powder, solid, soluble form such as lotion, emulsion form such as emulsion, cream and paste, ointment and suspension.

The agent of the present invention means an external agent applied to outer skin such as skin and scalp and oral cavity as a cosmetic, medicine or quasi drug, for example, ointment, cream, emulsion, lotion, essential, jelly, pack, mask, bath agent, dental powder, dental gel, dental paste, dental liquid, dental medicine, mouth freshener, mouth freshening film, mouthwash and gargle, including composition for oral administration such as chewing gum and candy for keeping firmness, elasticity and color of gum-ridge.

The following experiments explain the efficacy of the indigo-plant extract of the present invention in details. In the following experiments, the indigo-plant extract not containing 1,3-butyleneglycol, which is prepared by the method of Example 1 described later unless specified otherwise.

Experiment 1

Effect of extract of polygonaceous plants on melanin production (in vitro test 1 for skin-whitening effect)

The following experiment was carried out to investigate the effect of extract of polygonaceous plants on melanin production. After $2.2 \times 10^5$ of B16 melanoma cells of mouse were cultured by conventional method for 24 hours, the medium was added with indigo-plant extract to give a concentration of 1.25% or 2.5%, then they were cultured for additional 72 hours. After the number of the living cells was counted, color of the cells packed in capillary was observed. Using a medium added with any one of the extract of Japanese knotweed, buckwheat and knotgrass prepared by the same method of Example 1 described later to give a concentration of 2.5%, or added with koji acid to give a concentration of 2.5 mM as control, the B16 melanoma cells were cultured by the same manner as using indigo-plant extract. Then the colors of the cells were observed by the same manner as using indigo-plant extract. The degree of blackening of B16 melanoma cells was evaluated by the criterion described below. The results are shown in Table 1.

The criterion for evaluation of blackening:
Efficacy compared to the control added with no agent
-: Not changed
±: Slightly suppressing blackening
+: Moderately suppressing blackening
++: Significantly suppressing blackening
+++: Completely suppressing blackening

Table 1

| Composition of medium | Efficacy |
|---|---|
| Medium alone | - |
| Medium containing 1.25% of indigo-plant extract | + |
| Medium containing 2.5% of indigo-plant extract | ++ |
| Medium containing 2.5% of Japanese knotweed extract | ± |
| Medium containing 2.5% of buckwheat extract | ± |
| Medium containing 2.5% of knotgrass extract | ± |
| Medium containing 2.5 mM of koji acid | + |

As is evident from Table 1, the cells cultured in the medium containing 1.25% or 2.5% of indigo-plant extract exhibited the same or higher effect in suppressing melanin production than the cells cultured in the medium containing 2.5 mM of koji acid as control. When the same concentration of extract of Japanese knotweed, buckwheat and knotgrass were used, the blackening was suppressed little. These results indicate that extract of indigo-plant among polygonaceous plants exerts extremely strong suppressing effect on melanoma production by directly affecting on melanocyte. At the above concentration of the indigo-plant extract, no cytotoxicity was observed. As indigo-plant extract does not inhibit tyrosinase activity, the suppression of melanin production by indigo-plant extract is considered to be due to the suppression of the synthesis of tyrosinase. The indigo-plant extract used in Experiment 1 contained tryptanthrin at the final concentration of 0.33 µg/ml. When 0.5 µg/ml or more of tryptanthrin was used instead of the indigo-plant extract (no cytotoxicity to B16 melanoma cells was observed at a concentration of 0.5 µg/ml), melanin production was suppressed although the effect was lower than the one of indigo-plant extract. These results indicate that one of the effective substances for skin-whitening is tyrptanthrin.

Experiment 2

Effect of indigo-plant extract and other skin-whitening ingredient on melanin production (*in vitro* test 2 for skin-whitening effect)

The following experiment was carried out to investigate the skin-whitening effect of indigo-plant extract and other skin-whitening ingredient. After B16 melanoma cells of mouse were cultured for 24 hours, the test samples were added to the medium alone or along with 1.25% of the indigo-plant extract to give the concentrations described in Table 2, the cells were cultured for additional 72 hours. After the number of the living cells was counted, a color of the cells packed in capillary was observed. The results were shown in Table 2. The effect was evaluated on the same criterion in Experiment 1. When the experiment was conducted using 1.25% of the indigo-plant extract, the effect was evaluated as "+".

Table 2

| Skin-whitening ingredient | Content (%) | Efficacy | |
|---|---|---|---|
| | | Without indigo-plant extract | With 1.25% of indigo-plant extract |
| Sodium L-ascorbate | 3.0 | + | +++ |
| Potassium 4-methoxysalicylate | 1.0 | + | +++ |
| Koji acid | 1.0 | + | +++ |
| Arbutin | 5.0 | + | +++ |
| Tranexamic acid | 1.0 | ± | ++ |
| Ellagic acid | 0.5 | ± | ++ |
| 4-n-Butylresorcinol | 3.0 | + | +++ |
| Linoleic acid | 0.5 | ± | ++ |
| Camomile extract* | 1.0 | + | +++ |
| Tetrahydrocurcumin | 0.5 | ± | ++ |
| *:Extracted with ethanol/water (8/2; w/w) (the content was based on dry solid) | | | |

As evident from Table 2, every test sample containing sodium L-ascorbate, potassium 4-methoxysalicylate, koji acid, arbutin, tranexamic acid, ellagic acid, 4-n-butylresorcinol, linoleic acid, camomile extract and tetrahydrocurcumin exhibits suppressing effect on blackening synergistically when used together with the indigo-plant extract.

Experiment 3

Effect of indigo-plant extract and other skin-whitening ingredient on melanin production (in vivo test for skin-whitening effect )

The following experiment was carried out to investigate the skin-whitening effect of indigo-plant extract and other skin-whitening ingredient. Eleven group, one group was consists of 20 subjects who have dark skin, fleck or freckle, were applied with any one of the test lotions prescribed as follows on the face every morning and evening for three months to investigate the efficacy of skin-whitening.

(Recipe for the test lotions)

The solution dissolving with ingredient (3), (4) and (9) to (11) described below and the solution dissolving ingredients (1), (2), (5), (6), (7), (8) and (11) were homogeneously mixed to give the test lotions. As the "other skin-whitening ingredient" of (6), one of the ingredients described in Table 3 was added at the content described in Table 3. The efficacy of skin-whitening was evaluated using these test lotions. The results were shown in Table 3.

(Formula for preparation of the test lotions) (the numbers behind ":" means "%")

(1) Glycerol: 5
(2) 1,3-Butyleneglycol: 6.5
(3) Polyoxyethylene(20E.O.)sorbitanmonolauric acid ester: 1.2
(4) Ethyl alcohol
(5) Indigo-plant extract
(6) Other skin-whitening ingredient (described in Table 3): described in Table 3
(7) Lactic acid: 0.05
(8) Sodium lactate: 0.1
(9) p-Methoxycinnamic acid 2-ethylhexyl: 0.1
(10) Preservative: adequate amount
(11) Perfume: adequate amount
(12) Purified water: Residual amount

For comparison, the comparative lotions were prepared in accordance with the same recipe in Experiment 3 except for containing other skin-whitening ingredient (6) described in Table 3 in an amount described in Table 3 without indigo-plant extract (5). The efficacy of skin-whitening of these lotions was evaluated by the same way in Experiment 3. The results were shown in Table 3

Table 3

| | Other skin-whitening ingredient | Amount of other skin-whitening ingredient (%) | Amount of indigo-plant extract | Efficacy |
|---|---|---|---|---|
| Test lotion 1 | L-Ascorbic acid 2-glucoside | 0.5 | 0.5 | A |
| Test lotion 2 | Potassium 4-methoxysalicylate | 0.5 | 0.5 | A |
| Test lotion 3 | Koji acid | 0.5 | 0.5 | A |
| Test lotion 4 | Arbutin | 0.5 | 0.5 | A |
| Test lotion 5 | Tranexamic acid | 0.5 | 0.5 | B |
| Test lotion 6 | Ellagic acid | 0.5 | 0.5 | B |
| Test lotion 7 | 4-n-Butylresorcinol | 0.5 | 0.5 | A |
| Test lotion 8 | Linoleic acid | 0.5 | 0.5 | B |
| Test lotion 9 | Camomile extract | 0.5 | 0.5 | B |
| Test lotion 10 | Tetrahydrocurcumin | 0.5 | 0.5 | B |
| Comparative lotion 1 | Indigo-plant extract | 0 | 1 | B |
| Comparative lotion 2 | L-Ascorbic acid 2-glucoside | 1 | 0 | B |
| Comparative lotion 3 | Potassium 4-methoxysalicylate | 1 | 0 | C |
| Comparative lotion 4 | Koji acid | 1 | 0 | C |
| Comparative lotion 5 | Arbutin | 1 | 0 | C |
| Comparative lotion 6 | Tranexamic acid | 1 | 0 | C |
| Comparative lotion 7 | Ellagic acid | 1 | 0 | B |
| Comparative lotion 8 | 4-n-Butylresorcinol | 1 | 0 | C |
| Comparative lotion 9 | Linoleic acid | 1 | 0 | C |

(continued)

|  | Other skin-whitening ingredient | Amount of other skin-whitening ingredient (%) | Amount of indigo-plant extract | Efficacy |
|---|---|---|---|---|
| Comparative lotion 10 | Camomile extract | 1 | 0 | C |
| Comparative lotion 11 | Tetrahydrocurcumin | 1 | 0 | C |

The criterion and evaluation on the skin-whitening efficacy of the test lotions and comparative lotions are described as follows:

(Criterion)

Sufficiently effective: Almost disappearance of pigmentation
Effective: Sufficient decrease of pigmentation
Slightly effective: slight decrease of pigmentation
Not effective: No changing

(Evaluation)

A: Sufficiently effective or effective on 80% or more of the subjects
B: Sufficiently effective or effective on 50% or more and less than 80% or more of the subjects
C: Sufficiently effective or effective on 30% or more and less than 50% or more of the subjects
D: Sufficiently effective or effective on less than 30% of the subjects

As evident from Table 3, when the test lotion (agent for external application to the skin) containing any one of L-as corbie acid 2-glucoside, potassium 4-methoxysalicylate, koji acid, arbutin, trenexamic acid, ellagic acid, 4-n-butylresorcinol, linoleic acid, camomile extract and tetrahydrocurcumin along with the indigo-plant extract was used, the efficacy of skin-whitening was evaluated as A or B. On the other hand, when the comparative lotion containing any one of L-ascorbic acid 2-glucoside, potassium 4-methoxysalicylate, koji acid, arbutin, trenexamic acid, ellagic acid, 4-n-butylresorcinol, linoleic acid, camomile extract and tetrahydrocurcumin alone was used, the efficacy of skin-whitening was evaluated as B or C, in which the level of the skin-whitening efficacy was considered one step lower than the lotion containing indigo-plant extract. These results indicate that the agents for external application to the skin containing indigo-plant extract and other skin-whitening ingredient exerts excellent efficacy of the skin-whitening synergistically.

Experiment 4

Effect of extract of polygonaceous plant on elastase activity

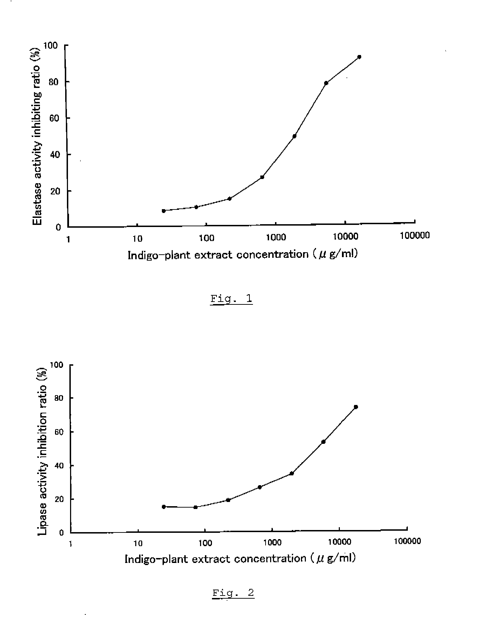

The following experiment was carried out to investigate the effect of extract of polygonaceous plant on elastase activity. By consecutive 3-fold dilution of indigo-plant extract (solid content of 1.41%) with purified water, the test samples of indigo-plant extract of solid content of 14,100 $\mu$g/ml, 4,700 $\mu$g/ml, 1,570 $\mu$g/ml, 520 $\mu$g/ml, 170 $\mu$g/ml, 60 $\mu$g/ml and 19 $\mu$g/ml were prepared. Fifty $\mu$l of each above sample was addmixed with 25 $\mu$l of 0.2 M Tris-HCl buffer (pH 8.5, containing 1% of BSA and 1 M NaCl) containing 20 $\mu$M of synthetic substrate, N-Methylsuccinyl-L-Alanyl-L-Alanyl-L-Prolyl-L-Valine-4-Methyl-Coum aryl-7-Amide (commercialized by Peptide Institude, Inc.) and 25 $\mu$l of 0.2 M Tris-HCl buffer containing 0.048 unit/ml of human neutrophil elastase, and incubated at 37°C for 60 minutes. Then, the amount of 4-nitroaniline produced by the enzyme reaction was determined by measurement of fluorescent intensity (Ex 355 nm, Em 460 nm) with fluorescent microplate Reader Fluoroscan II (BioTek instruments, Inc.). The elasitase activity inhibiting ratio was calculated by the following formula. The results were shown in Fig. 1.

$$\text{Inhibiting ratio (\%)} = \{(A-B)/A\} \times 100$$

A: Fluorescent intensity without adding test sample
B: Fluorescent intensity with adding test sample

As is indicated in Fig. 1, inhibitory effect on elastase activity was observed in the indigo-plant extract depending on the concentration. The same test was carried out using the knotweed extract (solid content of 1.65%), buckwheat extract (solid content of 1.37%) and knotgrass extract (solid content of 1.81%) used in Experiment 1 and its 50%-inhibitory concentration on elastase activity were compared to the indigo-plant extract. The inhibitory concentration of the indigo-plant extract was 1.11 mg/ml. However the inhibitory efficacy of the extracts of knotweed, buckwheat and knotgrass were 12%, 13% and 25%, respectively, when used these extract without dilution and these 50%-inhibitory concentrations can not be obtained. These results indicate that indigo-plant extract has strong inhibitory effect on elastase activity compared to other polygonaceous plants, knotweed, buckwheat and knotgrass and that the agent for external application to the skin containing indigo-plant extract exhibits excellent suppressing effect on forming wrinkle.

Experiment 5

Effect of indigo-plant extract on lipase activity

The following experiment was carried out to investigate the effect of indigo-plant extract on lipase activity. Twenty five $\mu$l of any one of indigo-plant extract used in Experiment 4 and 50 $\mu$l of McIlvaine buffer (pH 7.4, consisting of 9.15% of 0.1 M citric acid and 90.85% of 0.2 M $Na_2HPO_4$) containing 0.1 M 4-Methylumbelliferylolleate were admixed and the solution was added with 25 $\mu$l of McIlvaine buffer containing 1 unit/ml of lipase, then reacted at 37°C for 20 minutes. After reacted, the solution was added with 50 $\mu$l of 1N HCl and 100 $\mu$l of sodium citrate to stop the reaction, and the amount of produced 4-Methylumbelliferon were determined by measurement of the fluorescent intensity (Ex 355 nm, Em 460 nm) with fluorescent microplate reader Fluoroscan II (BioTek instruments, Inc.). The lipase activity inhibiting ratio was calculated by the following formula. The results were shown in Fig. 2.

$$\text{Inhibiting ratio (\%)} = \{1-(A-B)/(C-B)\} \times 100$$

A: Fluorescent intensity with adding test sample
B: Fluorescent intensity of McIlvaine buffer
C: Fluorescent intensity without adding test sample

As is shown in Fig. 2, indigo-plant extract has an inhibitory effect on lipase activity depending on its concentration. This result indicates that the agent for external application to the skin has excellent suppressing effect on acne.

Experiment 6

Effect of indigo-plant extract on SOD mimicking activity

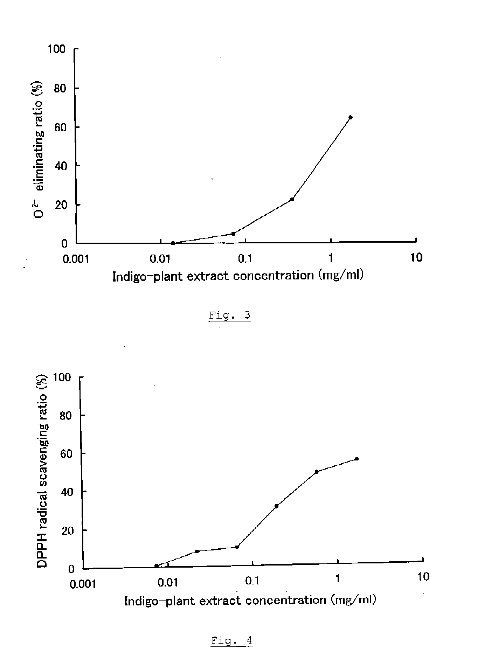

The following experiment was carried out to investigate the effect of indigo-plant extract on SOD mimicking activity. The experiment was conducted by using SOD Test Wako (Wako Pure Chemical Industries, Ltd.) which is based on NBT (nitro blue tetrazolium) reduction method.
Ten $\mu$l of any one of the indigo-plant extract and its dilutions used in Experiment 4 and 95 $\mu$l of the coloring reagent containing 0,24 M NBT and 0.4 M xanthine were admixed and the solution was added with 95 $\mu$l of the enzyme solution (xanthine oxidase), then reacted at 37°C for 30 minutes. After the reaction was stopped by addition of 100 $\mu$l of the reaction terminating agent (69 mM sodium dodecylsulfate), absorbance at a wavelength of 562 nm was measured. Using the samples added with purified water instead of enzyme solution as blank and the samples added with the solvent instead of the test extracts as control, the superoxide anion ($O^{2-}$) eliminating ratio was calculated by the following formula. The results were shown in Fig. 3.

$$\text{Eliminating ratio (\%)} = \{(A-B)/A\} \times 100$$

A: Absorbance of control
B: Absorbance without adding test sample

As is shown in Fig. 3, indigo-plant extract has SOD mimicking activity (superoxide anion eliminating activity) depending on its concentration. This result indicates that the agent for external application to the skin has excellent effect in antiaging.

Experiment 7

Effect of indigo-plant extract on 1,1-diphenyl-2-picrylhydrazyl radical

The following experiment was carried out to investigate the effect of indigo-plant extract on 1,1-diphenyl-2-picryl hydrazyl radical (hereinafter, abbreviated as "DPPH"). By consecutive 3-fold dilution of indigo-plant extract (solid content of 1.41%) with 5 mM acetate buffer (pH 5.5), the test samples of indigo-plant extract of solid content of 14,100 $\mu$g/ml, 4,700 $\mu$g/ml, 1,570 $\mu$g/ml, 520 $\mu$g/ml, 170 $\mu$g/ml, 60 $\mu$g/ml and 19 $\mu$g/ml were prepared. Fifty $\mu$l of each above sample was admixed with 100 $\mu$l of DPPH ethanol solution. After the solutions were reacted at a room temperature for 20 minutes, the absorbance at a wavelength of 515 nm was measured with microplate reader Model EL-340 (BioTek instruments, Inc.). The level of activity was represented by the DPPH radical scavenging ratio obtained by the following formula. The results were shown in Fig. 4.

$$\text{Scavenging ratio (\%) = \{1-(A-B)/(C-B)\} \times 100}$$

A: Fluorescent intensity with adding test sample
B: Fluorescent intensity of acetate buffer
C: Fluorescent intensity without adding test sample

As is shown in Fig. 4, indigo-plant extract has DPPH radical eliminating activity depending on its concentration. These results indicate that the agent for external application to the skin has excellent effect in antiaging.

Experiment 8

Effect of indigo-plant extract on skin cell

As is evident from Experiments 4 to 7, it was found that indigo-plant extract have inhibitory effect on the activity of the enzymes which may influence the aging of the skin or wrinkle formation for human as well as skin-whitening effect. Then, the effect of indigo-plant extract on human skin was ascertained in panel test with volunteer subjects. Thirty-five parts by weight of carboxyvinyl polymer solution of 2%, five parts by weight of conc. glycerol, 1.8 parts by weight of sodium hydroxide solution of 10%, three parts by weight of pentyleneglycol and adequate amount of the indigo-plant extract (solid content of 1%, not containing 1,3-butyleneglycol) which is prepared by the method in Example 1 described later were admixed with adequate amount of purified water to give the test gels with the amount of the indigo-plant extract described in Table 4. A control gel was prepared in the same recipe as the above using water instead of the 50 parts by weight of the indigo-plant extract. Sixth subjects were randomly separated into 6 groups consisting of 10 subjects each. Ten subjects of each group were applied with any one of test gels containing certain concentration of indigo-plant extract and the control gel to each different area of the medial side of the arm the different parts three times a day for 56 days. At the day of the last application the area of being applied with gel were irradiated with ultraviolet, which dose was twice of the minimum erythema dose (the minimum dose inducing erythema at irradiated area of each subject) determined by irradiation of various doses of ultraviolet. After 24 hours from the irradiation, visual check of the level of erythema formation and manipulation test of the level of change in skin elasticity were carried out on each area of application of the test gel and the control gel. The criterion and evaluation of efficacy of gel application was conducted by the method described below. The results were shown in Table 4.

(Criterion)

<erythema>

Sufficiently effective: little erythema was formed when applied with test gel
Effective: erythema formation was suppressed compared to when applied with test gel
Not effective: the same level of etythema formation was observed as applied with control gel
Getting worse: erythema formation was enhanced compared to when applied with test gel

<elasticity>

Sufficiently effective: elasticity was increased with elevated skin thickness when applied with test gel

Effective: elasticity was increased without elevated skin thickness when applied with test gel
Not effective: the same level of elasticity was observed as applied with control gel
Getting worse: elasticity was reduced compared to when applied with test gel

(Evaluation)

A: Sufficiently effective or effective on 80% or more of the subjects
B: Sufficiently effective or effective on 50% or more and less than 80% or more of the subjects
C: Sufficiently effective or effective on 30% or more and less than 50% or more of the subjects
D: Sufficiently effective or effective on less than 30% of the subjects

Table 4

| Content of indigo-plant extract (%) | Efficacy | |
| --- | --- | --- |
| | Erythema formation | Skin elasticity |
| 50 | A | A |
| 5 | A | A |
| 0.5 | A | B |
| 0.05 | B | B |
| 0.001 | C | C |

As evident from Table 4, when the indigo-plant extract was added to gel at a concentration of 0.05% (0.0005% or more on a dry solid basis) or more, level of erythema formation and elasticity were evaluated as B, which means the effect of gel application was observed on half or more of subjects, compared to the control gel. The efficacy increased when added at 0.5% (0.005% or more on a dry solid basis) or more, and increased much more as evaluated as A when added at 5% (0.05% or more on a dry solid basis) or more.

Experiment 9

Effect of indigo-plant extract on skin cell

As evident from the results in Experiment 8, it was found that the gel containing indigo-plant extract at 50% used in Experiment 8 suppressed erythema formation by ultraviolet irradiation on human skin and significantly increased skin elasticity. To ascertain the above efficacy, the skin applied with indigo-plant extract was histologically analyzed. Thirty-five parts by weight of carboxyvinyl polymer solution of 2%, five parts by weight of conc. glycerol, 1.8 parts by weight of sodiumhydroxide solution of 10%, three parts by weight of pentyleneglycol, 50 parts by weight of the indigo-plant extract (solid content of 1%, containing 1,3-butyleneglycol at 30%) which is prepared by the method in Example 1 described later and 5.2 parts by weight of purified water were admixed to give the test gel. A control gel was prepared in the same recipe as the above using 15 parts by weight of 1, 3-butyleneglycol instead of the 50 parts by weight of the indigo-plant extract and 40.2 parts by weight of water. The five volunteer subjects were applied with the test gel or the control gel to each different area of the medial side of the arm the different parts three times a day for 56 days. At the day of the last application the area of being applied with gel were irradiated with ultraviolet, which dose was twice of the minimum erythema dose (the minimum dose inducing erythema at irradiated area of each subject) determined by irradiation of various doses of ultraviolet, as is in Experiment 8. After 24 hours from the irradiation, the skin tissues were aseptically extracted each from ultraviolet-irradiated area applied with the test gel or the control gel and not applied with either gel with biopsy punch under xylocaine anesthesia. The extracted cell tissues were fixed with formalin and embedding in paraffin by conventional way, then the prepared tissue slice was analyzed histologically. The tissue slice was used for measurement of the thickness of epidermal layer and dermal layer, the number of sunburned cells (dead cells) caused by ultraviolet irradiation and observation of elastogenesis in the dermal layer by Elastica-van Gieson method. The thickness of epidermal layer and dermal layer were determined as follows: Thirty points of the tisshue slice was randomly selected by using a light microscope and the images of them were taken with a digital camera; thickness of the layer at these points were measured with a slide gauge and calculated the average of them; the increasing rate (%) of the thickness of the dermal layer was calculated by dividing the average of the thickness of the area applied with test gel or control gel by the average of the thickness of the area applied with no gel. The average of the increasing rate of the five volunteers were calculated and shown in Table 5. The number of the sunburn cells was measured as follows: the images of the 30 points of the tissue slice randomly selected were taken with a digital camera; the number of the sunburned cells was counted and the average of them was calculated; the increasing rate (%) of the sunburned cell was calculated by dividing the average of the number of sunburned cells at the area applied with test gel or control gel by the average of the number of sunburned cells of the area applied with no gel. The average of the increasing rate of the five volunteers were calculated and shown in Table 5.

Table 5

| Applied gel | Measurement item | | |
|---|---|---|---|
| | Increasing rate of epidermal layer (%) | Increasing rate of dermal layer (%) | Increasing rate of sunburned cell (%) |
| Test gel | 138 | 139 | 119 |
| Control gel | 121 | 124 | 147 |

As is evident from Table 5, the thickness of epidermal layer and dermal layer and the number of sunburned cell were increased at the area applied with test gel or control gel compared to one applied with no gel. The thickness of epidermal layer and dermal layer of the area applied with test gel was higher and the number of sunburned cells of the area applied with test gel was lower than the area applied with control gel. Microscopic observation of the elastic fiber showed regular array of the fiber on the area applied with no gel, and the irregular array of the fiber on the area applied with control gel. On the other hand, elastic fibers were increased throughout the dermal layer on the area applied with test gel. There results indicate that the indigo-plant extract can be used as effective antiaging agent, since skin cells was prevented from damage by ultraviolet irradiation as well as the level of skin elasticity was elevated by increasing the turnover of epidermal layer and dermal layer when the indigo-plant extract was used as the agent for external application to the skin.

Experiment 10

Effect of indigo-plant extract on human dental bacteria

The following experiment was carried out to investigate the effect of indigo-plant extract on human dental bacteria. Two parts by weight of shredded whole indigo plant was added with three parts by weight of purified water and stirred at a room temperature for 10 minutes to prepare the extract. The supernatant obtained by centrifugation was autoclaved at 120°C for 20minutes. After autoclaved, the supernatant obtained by centrifugation was filtrated to give an indigo-plant extract (solid content of 1.41%). The minimum inhibitory concentration (hereinafter, occasionally abbreviated as "MIC") of the extract to dental bacteria (periodontal disease bacteria and caries bacteria) was determined. Also MIC of tryptanthrin, gallic acid and caffeic acid, which are found to be contained in the indigo-plant extract to dental bacteria. These results were shown in Table 6. The measurement of MIC was conducted by the method described below.

<Measurement of MIC>

MIC of the indigo-plant extract, tirptanthrin, gallic acid and caffeic acid was determined according to the standard method of Japanese society of Chemotherapy (q.v. "Saikin-Shinkin Kensa" (Second edition), issued by Japan Public Health Association, pp.524-529 (1982)). For periodontal disease bacteria, *Prophyromonas gingivalis* JCM8525, JCM12257 and ATCC33277, *Actinobacillus actinomycetemcomitans* (hereinafter, occasionally abbreviated as "A. *actinomycetemcomitans*") JCM2434 and JCM8577, *Prevotella intermedia* (hereinafter, occasionally abbreviated as "*P. intermedia*") JCM7365, and *Campylobacter rectus* (hereinafter, occasionally abbreviated as "C. *rectus")* JCM6301 were used. For caries bacteria, Streptococcus mutans OMZ-175, OMZ-176, OMZ-65, OMZ-61, B-13D and Ingbritt, and Streptococcus *sorbrinus* (hereinafter, occasionally abbreviated as *"S. sorbrinus")* 6715 were used. These bacteria were anaerobically cultured in Brain Heart Infusion medium (commercialized by Difco Laboratories Inc., hereinafter abbreviated as "BHI") at 37°C for 48 hours for periodontal disease bacteria or for 18 to 24 hours for caries bacteria. The broth of periodontal disease bacteria and caries bacteria was diluted to give the bacteria suspensions containing $10^8$ cells/ml and $10^6$ cells/ml, respectively. Five $\mu$l of any one of these suspension was inoculated on BHI agar plate containing various concentration of the indigo-plant extract, tryptanthrin, gallic acid or caffeic acid and anaerobically cultured at 37°c for 24 hours. Then, MIC was obtained as the concentration of the above ingredients at which the bacterial growth was completely suppressed.

Table 6

| Dental bacteria | | Minimum Inhibitory Concentration (MIC) | | | |
|---|---|---|---|---|---|
| | | Indigo-plant extract | Tryptanthrin | Gallic acid | Caffeic acid |
| | | (mg/ml) | ($\mu$g/ml) | | |
| A. actinomycetemcomitans | JMC12257 | 1.74 | 12.5 | 400 | 400 |
| | JMC8525 | 1.74 | 6.25 | 400 | 400 |
| | JMC33277 | 1.74 | 12-5 | 400 | 400 |
| | JMC2434 | 3.84 | 25 | 800 | 400 |
| | JMCB577 | 3.84 | 25 | 800 | 200 |
| C. rectus | JMC6301 | 1.74 | 25 | 400 | 100 |
| P. intermedia | JMC7365 | 1.74 | 6.25 | 400 | 400 |
| S. mutans | OMZ-176 | 3.84 | 12.5 | 400 | 800 |
| | OMZ-175 | 3.84 | 6.25 | 400 | 400 |
| | OMZ-65 | 3.84 | 12.5 | 1600 | 200 |
| | B-13D | 1.74 | 6.25 | 200 | 200 |
| | Ingbritt | 3.84 | 25 | 1600 | 1600 |
| | OMZ-61 | 1.74 | 6.25 | 200 | 400 |
| S. sobrinus | 6715 | 3.84 | 25 | 800 | 800 |

As is evident from Table 6, indigo-plant extract has strong antimicrobial activity on seven strains in four species of periodontal disease bacteria and seven strains in two species with MIC of 1.74 mg/ml to 3.48 mg/ml on a dry solid basis. Also tryptanthrin had strong antimicrobial activity and gallic acid and caffeic acid had small antibacterial activity with MIC of 6.25 $\mu$g/ml to 25 $\mu$g/ml, 200 $\mu$g/ml to 1600 $\mu$g/ml and 200 $\mu$g/ml to 1600 $\mu$g/ml, respectively. Caffeic acid had no antimicrobial activity. Although the indigo-plant extract used in this experiment contains tryptanthrin, gallic acid and caffeic acid at a concentration of about 0.51 $\mu$g/ml, about 0.55 $\mu$g/ml and about 1.07 $\mu$g/ml, respectively, sufficient MIC can not be obtained at these concentrations to periodontal disease bacteria and caries bacteria. This result suggested that the antimicrobial activity of indigo-plant extract is due to also other ingredients except tryptanthrin, gallic acid and caffeic acid. The growth-suppressing effect of the above agent on the bacteria in oral cavity used for MIC measurement was observed at about 1/100 concentration or more of each MIC and the efficacy increased depending on its concentration.

<Antiseptic effect>

The following experiment was carried out to ascertain the antiseptic effect of indigo-plant extract on *Prophyromonas gingivalis* JCM8525. The same indigo-plant extract as used for MIC measurement was added to BHI at a concentration of 6.95 mg/ml, 3.48 mg/ml or 1.74 mg/ml, on a dry solid basis, then the living cells of *Prophyromonas gingivalis* JCM8525 was added to the medium at a concentration of $10^8$ cells/ml and cultured at 37°C for 3 or 9 hours. These broths were accordingly diluted and inoculated to BHI agar plate. After cultured at 37°C for 24hours, the number of living cells before dilution was determined by counting the formed colony. As the control, the number of living cells in broth cultured in BHI added with deionized water instead of the indigo-plant extract was determined by the same way described above.

When cultured in the medium added with 6.95 mg/ml of the indigo-plant extract, Prophyromonas *gingivalis* JCM8525 was completely killed in the 3 hours. When cultured in the medium added with 3.48 mg/ml of the indigo-plant extract, the number of the living cells was reduced to 1/1000 for 3 hours, to 1/10000 for 9 hours. When cultured in the medium added with 1.74 mg/ml of the indigo-plant extract, the number of the living cells was reduced to 1/10 for 3 hours, to 1/100 for 9 hours. The number of the living cells of *Prophyromonas gingivalis* decreased depending on the concentration of indigo-plant extract and culturing period. On the other hand, when cultured without addition of the indigo-plant extract, the number of the living cells did not decreased. These results indicated that indigo-plant extract has high antimicrobial effect on *Prophyromonas gingivalis.*

The above experimental results indicated that since the indigo-plant extract of the present invention exhibits all effects in suppressing melanin production, inhibiting elastase activity, inhibiting lipase activity, eliminating active oxygen, scavenging radical, increasing turnover of epidermal or dermal cells and preventing cells from ultraviolet hazard, the agent for external application to the skin comprising indigo-plant extract, which can suppress fleck and freckle formation and pigmentation after sunburn, has excellent effects in skin-whitening, skin-beautifying and antiaging. They also indicated that when used as an external agent for oral cavity, it exhibits preventive or therapeutic effect on periodontal disease and caries.

The present invention was explained by following examples, which do not restrict the present invention.

Example 1

Preparation of indigo-plant extract

Fifteen parts by weight of shredded whole fresh indigo-plant was added with 23 parts by weight of purified water and stirred at room temperature for 10 minutes to prepare its extract, and then the supernatant obtained by centrifugation was autoclaved. After autoclaved, the solution was centrifuged and filtrated to give an indigo-plant extract. Since the extract has various physiological activities such as suppressing melanin production, inhibiting elastase activity, inhibiting lipase activity, suppressing sebum secretion from grandula sebacea, SOD mimicking activity, scavenging DPPH radical and antiaging, it can be advantageously used as an effective substance for skin-whitening and/or skin-beautifying agent for external application to the skin.

Table 7 shows the appearance property, pH, colorability (OD 420 nm) and turbidity (OD 720 nm) of two-fold dilution, amount of polyphenols and amount of dry solid of the extract.

Table 7

| Item of analysis | |
|---|---|
| Appearance property | Brownish-red fluid |
| pH | 4.87 |
| Colorability (OD 420 nm) | 1.168 |
| Turbidity (OD 720 nm) | 0.028 |
| Amount of polyphenols ($\mu$g/ml) | 453 |
| Amount of dry solid (%) | 1.41 |

Seven parts by weight of the extract was added with 1,3-butyleneglycol (hereinafter, abbreviated as "1.3-BG") as preservative to give an indigo-plant extract. This extract has high storage stability because of suppressed sediment and odor formation and browning compared to the extract without 1.3-BG. Since the extract has various physiological activities such as suppressing melanin production, inhibiting elastase activity, inhibiting lipase activity, suppressing sebum secretion from grandula sebacea, SOD mimicking activity, scavenging DPPH radical and antiaging, it can be advantageously used as an effective substance for skin-whitening and/or skin-beautifying agein for external application to the skin.

Example 2

Preparation of indigo-plant extract

Four parts by weight of shredded whole fresh indigo-plant was added with 3 parts by weight of mixture of equal part of purified water and ethanol and stirred for 20 minutes. Then the supernatant obtained by centrifugation was autoclaved to give an indigo-plant extract. Since this extract is an indigo-plant extract with suppressed sediment and odor formation and browning and has various physiological activities such as suppressing melanin production, inhibiting elastase activity, inhibiting lipase activity, suppressing sebum secretion from grandula sebacea, SOD mimicking activity, scavenging DPPH radical and antiaging, it can be advantageously used as an effective substance for skin-whitening and/or skin-beautifying agent for external application to the skin.

Example 3

Preparation of indigo-plant extract

The indigo-plant extract prepared by the method of Example 1 (not containing 1,3-BG) was ultrafiltrated to give an indigo-plant extract. This extract exhibits low formation of sediment or turbidity in longtime storage and since it has elevated (about 100 fold) permeability through a membrane of pore size of 0 - 45 $\mu$m compared to not ultrafiltrated extract by removable of small particles, it is an easy-handled indigo-plant extract as can be sterilized by filtration. Since this extract is an indigo-plant extract with suppressed odor formation and browning and has various physiological activities such as suppressing melanin production, inhibiting elastase activity, inhibiting lipase activity, suppressing sebum secretion from grandula sebacea, SOD mimicking activity, scavenging DPPH radical and antiaging, it can be advantageously used as an effective substance for skin-whitening and/or skin-beautifying agein for external application to the skin.

Example 4

Preparation of powdery indigo-plant extract

An amount equal to one part by weight of the dry solid of the indigo-plant extract prepared by the method of Example 3 was admixed with one part by weight of "Isoelite P" (branched cyclodextrin commercialized by Ensuiko Sugar Refining Co., Ltd.) and 0.1 parts by weight of $\alpha,\alpha$-trehalose, on a dry solid basis, dissolved by stirring and lyophilized by conventional method to give a powdery indigo-plant extract. Since this extract is an indigo-plant extract with elevated level of preservative stability as not to turn brownish after longtime storage and has various physiological activities such as suppressing melanin production, inhibiting elastase activity, inhibiting lipase activity, suppressing sebum secretion from grandula sebacea, SOD mimicking activity, scavenging DPPH radical and antiaging, it can be advantageously used as an effective substance for skin-whitening and/or skin-beautifying agent for external application to the skin.

Example 5

Preparation of powdery indigo-plant extract

An amount equal to one part by weight of the 10-fold concentrated indigo-plant extract prepared by the method of Example 3 was added with one part by weight of "Isoelite P" (branched cyclodextrin commercialized by Ensuiko Sugar Refining Co., Ltd.) and 0.1 parts by weight of syrup comprising sugar derivatives of $\alpha,\alpha$-trehalose, dissolved by stirring, and spray-dried by conventional method to give an powdery indigo-plant extract. Since this extract is an indigo-plant extract with elevated level of preservative stability as not to turn brownish after longtime storage and has various physiological activities such as suppressing melanin production, inhibiting elastase activity, inhibiting lipase activity, suppressing sebum secretion from grandula sebacea, SOD mimicking activity, scavenging DPPH radical and antiaging, it can be advantageously used as an effective substance for skin-whitening and/or skin-beautifying agent for external application to the skin.

Example 6

Cream

A cream was prepared by conventional method according to the following formula.
(Formula) (the numbers behind ":" means "%")
Polyglyceryl myristate-10: 3
Lanolin: 0.5
Octyldodecanol: 2
Cetyl octanate: 3
Squalene: 5
Dimethicone: 0.3
Behanyl alcohol: 3
Cetyl alcohol: 2
Buthyl alcohol: 1
Cetyl palmitate: 1.5
Glyceryl stearate: 2.3
Butyleneglycol: 5

Pentyleneglycol: 3.5
Stearic acid: 0.5
Glycerol: 5
"TORNARE" (a saccharide composition comprising saccharide derivatives of $\alpha,\alpha$-trehalose, commercialized by Hayashibara Biochemical Laboratories, Inc.): 2
Pullulan: 0.1
Indigo-plant extract prepared by the method of Example 1 (not containing 1,3-BG): 1
Water: 59.3

The product can be used as an agent for external application to the skin for skin-whitening, skin-beautifying and/or antiaging. This product also has excellent permeability into the skin and spreadability and can make the skin smooth and soft by pullulan contained in it.

Example 7

Gel

A gel was prepared by conventional method according to the following formula.
(Formula) (the numbers behind ":" means "%")
Indigo-plant extract prepared by the method of Example 1 (not containing 1,3-BG): 20
Hyaluronic acid: 0.25
Photosensitizer 201: 0.005
"MINERALTREHA" (abrine product commercialized by H+B Life Science Co., Ltd.): 1
$\alpha,\alpha$-trehalose: 4.5
Glycerol: 5
1,3-BG: 5
Ethanol: 5
"HIVIS Wako 104" (a carboxyvinylpolymer product commercialized by Wako Pure Chemical Industries, Ltd.): 0.64
Polyoxyethylene (20) monosorbitane monooleol: 1
2,2',2"-Nitrotriethanol: 1
Ethyl $p$-oxybenzoate: 0.1
Purified water: 56.9
The product can be used as an agent for external application to the skin for skin-whitening, skin-beautifying and/or antiaging. This product also has excellent permeability into the skin and spreadability and can make the skin smooth and soft by pullulan contained in it.

Example 8

Emulsion

An emulsion was prepared by conventional method according to the following formula.
(Formula) (the numbers behind ":" means "%")
Polyoxyethylene (20E.O.) polyoxyprolylene (2E.O.) cetyl alcohol: 1
Silicon KF96 (20cs, commercialized by Shin-Etsu Chemical Co., Ltd): 2
Liquid paraffin (medium viscosity): 3
1,3-BG: 5
4-n-butylresorcinol: 0.3
Glycerol: 2
Ethyl alcohol: 15
Carboxyvinylpolymer: 0.3
Hydroxypropylcellulose: 0.1
2-Aminomethylpropanol: 0.1
Indigo-plant extract prepared by the method of Example 1 (not containing 1,3-BG): 2
Preservative: adequate amount
Purified water was added to make total amount 100%.

The product can be used as an agent for external application to the skin for skin-whitening, skin-beautifying and/or antiaging. This product also has excellent permeability into the skin and spreadability and can make the skin smooth and soft by pullulan contained in it.

Example 9

Emulsion

An emulsion was prepared by conventional method according to the following formula.
(Formula) (the numbers behind ":" means "%")
Polyoxyethylene(20E.O.) polyoxyprolylene(2E.O.) cetyl alcohol: 1
Silicon KF96 (20cs, commercialized by Shin-Etsu Chemical Co., Ltd): 2
Liquid paraffin (medium viscosity): 3
1,3-BG: 5
L-Ascorbic acid 2-glucoside: 2
Glycerol: 2
Ethyl alcohol: 15
Carboxyvinylpolymer: 0.3
Hydroxypropylcellulose: 0.1
2-Aminomethylpropanol: 0.1
Indigo-plant extract prepared by the method of Example 1 (not containing 1,3-BG): 2
Preservative: adequate amount
Purified water was added to make total amount 100%.

The product can be used as an agent for external application to the skin for skin-whitening, skin-beautifying and/or antiaging. This product also has excellent permeability into the skin and spreadability and can make the skin smooth and soft by pullulan contained in it.

Example 10

Shampoo

A shampoo was prepared by conventional method according to the following formula.
(Formula) (the numbers behind ":" means "%")
Pyrocton-Olamin: 0.5
Disodium edentate: 0.3
Photosensitizer 201: 0.002
Citric acid: 0.3
Sodium salicylate: 0.2
1,3-BG: 3
Sodium polyoxyethylenelaurylsulfate: 6.75
Coconut oil fatty acid diethanolamide: 2
Triethanolamine laurylsulfate: 10
Polyoxyethylenelanolic acid(80E.O.): 0.5
2-Alkyl-N-carboxymethyl-N-hydroxyethylimidazolyniumbetaine: 10
Hydroxyethylcellulosehydroxypropyltrimethylammmoniumchloride ether: 0.8
"AI-LUROS" (an indigo-plant extract containing 30% of 1,3-BG, commercialized by Hayashibara Biochemical Laboratories, Inc.): 5
Perfume: 0.2
Purified water was added to make total amount 100%.
The product is a shampoo having excellent effect in hair-growing, effect in suppressing hair los and effect in keeping scalp clean due to its effect in anti-inflammation or antiaging on scalp and strong antimicrobial activity.

Example 11

Hair tonic

A shampoo was prepared by conventional method according to the following formula.
(Formula) (the numbers behind ":" means "%")
Phtosensitizer 301: 0.005
Hydrated crystalline $\alpha,\alpha$-trehalose: 0.03
"$\alpha$ GLUCOSYLHESPERIDIN" (a glycosylated hesperidin product commercialized by Hayashibara Biochemical Lablratories, Inc.): 0.01

Indigo-plant extract prepared by the method of Example 1 (containing 1,3-BG): 5
Ethanol: 45
Purified water was added to make total amount 100%.

The product is a hair tonic having excellent effect in hair-growing, effect in suppressing hair los and effect in keeping scalp clean due to its effect in anti-inflammation or antiaging on scalp and strong antimicrobial activity, when this product was used to scalp transplanted with hair root, it exhibits excellent effect in elevating graft survival because of growth acceleration of hair papilla cells.

Example 12

Dental paste

A dental paste was prepared by conventional method according to the following formula.
(Formula) (the numbers behind ":" means "%")
Dicalcium phosphate: 45
Pullulan: 2.9
Sodium laurylsulfate: 1.5
Glycerol: 20
Polyoxyethylenesorbitanlaurate: 0.5
Sorbitol: 10
Maltitol: 7
Indigo-plant extract prepared by the method of Example 1 (not containing 1,3-BG): 0.5
Tetrahydrocurcumin: 0.5
Preservative: adequate amount
Purified water was added to make total amount 100%.

Since the product contains indigo-plant extract, it has effect in keeping firmness and elasticity of gum-ridge and antiinflammation and can be used as a cosmetic for preserving and improving oral health. It can be advantageously used for anticaries and prevention and control the progress of periodontal disease because the antiseptic ingredients in indigo-plant extract exert bacteriostatic and antiseptic effect on caries bacteria and periodontal disease bacteria existing in tooth surface and periodontal pocket.

Example 13

Dental paste

A dental paste was prepared by conventional method according to the following formula.
(Formula) (the numbers behind ":" means "%")
β-Glycyrrhetininc acid: 0.05
Cetylpyridinium chloride: 0.05
Calcium hydrogen phosphate: 29
Indigo-plant extract prepared by the method of Example 1 (not containing 1,3-BG): 20
Conc. glycerol: 20
"TORNARE" (a saccharide composition comprising saccharide derivatives of $\alpha,\alpha$-trehalose, commercialized by Hayashibara Biochemical Laboratories, Inc.): 10
Anhydrous silicic acid: 5
Titanium oxide: 2
Sodium laurylsulfate; 1.2
Sodium carboxymethylcellulose: 1.2
Polyoxyethylene hydrogenated castor oil: 1
Ethanol: 0.5
Propolis extract: 0.5
Magnesium phosphate: 0.3
Sodium lauroylsarcosine: 0.2
Maltitol: 0.1
*p*-oxybenzoic acid ester: 0.1
Perfume: adequate amount
Purified water was added to make total amount 100%.

Since the product contains indigo-plant extract, it has effect in keeping firmness and elasticity of gum-ridge and anti-inflammation and can be used as a cosmetic for preserving and improving oral health. It can be advantageously used for anticaries and prevention and control the progress of periodontal disease because the antiseptic ingredients in indigo-plant extract exert bacteriostatic and anticeptic effect on caries bacteria and periodontal disease bacteria existing in tooth surface and periodontal pocket.

Example 14

Dental gel

A dental gel was prepared by conventional method according to the following formula.
(Formula) (the numbers behind ":" means "%")
β-Glycyrrhetininc acid: 0.05
Cetylpyridinium chloride: 0.05
Sorbitol solution: 30
Indigo-plant extract prepared by the method of Example 1 (not containing 1,3-BG): 10
Conc. glycerol: 10
"TORNARE" (a saccharide composition comprising saccharide derivatives of α,α-trehalose, commercialized by Hayashibara Biochemical Laboratories, Inc.): 10
Hydrous silicic acid: 9
Anhydrous silicic acid: 7
Xylitol: 3
Sodium carboxymethylcellulose: 1
Polyoxyethylene hydrogenated castor oil: 0.5
Ethanol: 0.5
Propolis extract (Commercialized by Hayashibara Biochemial laboratories, Inc.): 0.5
Maltitol: 0.1
*p*-oxybenzoic acid ester: 0.1
Perfume: adequate amount
Purified water was added to make total amount 100%.
Since the product contains indigo-plant extract, it has effect in keeping firmness and elasticity of gum-ridge and anti-inflammation and can be used as a cosmetic for preserving and improving oral health. It can be advantageously used for anticaries and prevention and control the progress of periodontal disease because the antiseptic ingredients in indigo-plant extract exert bacteriostatic and anticeptic effect on caries bacteria and periodontal disease bacteria existing in tooth surface and periodontal pocket.

Example 15

Mouthwash

A dental gel was prepared by conventional method according to the following formula.
(Formula) (the numbers behind ":" means "%")
Ethanol: 15
"HALLODEX" (a syrup comprising sugar derivatives of α,α-trehalose, commercialized by Hayashibara Biochemical Laboratories, Inc.): 8
Indigo-plant extract prepared by the method of Example 3: 2
"αG-HESPERIDIN" (a hesperidin glycoside commercialized by Hayashibara Biochemical Laboratories, Inc.): 1
Polyoxyethylene hydrogenated castor oil: 2
Sodium saccharin: 0.02
Sodium benzoate: 0.05
Sodium dihydrogen phosphate: 0.1
Colorant: adequate amount
Perfume: adequate amount
Filled up to make the total amount to 100%.
Since the product contains indigo-plant extract, it has effect in keeping firmness and elasticity of gum-ridge, suppressing xerostoma caused by Shogren's syndrome and preserving and improving anti-inflammation in oral cavity and dysgeusia, and has a good sense of use. It can be advantageously used for anticaries and prevention and control the progress of periodontal disease because the antiseptic ingredients in indigo-plant extract exert bacteriostatic and antiseptic effect on caries bacteria and periodontal disease bacteria existing in tooth surface and periodontal pocket.

Example 16

Ointment

An ointment was prepared by conventional method according to the following formula.
(Formula) (the numbers behind ":" means "%").
Sodium acetate: 1
Calcium phosphate: 4
Glycerol: 10
Mint oil: 0.5
Indigo-plant extract prepared by the method of Example 2: 3
L-Ascorbic acid 2-glucoside: 2
Vaseline: 49
Sumac wax: 10
Lanolin: 10
Sesame oil: 10.5
The product can be used as an agent for external application to the skin for skin-whitening, skin-beautifying and/or antiaging. This product also has excellent permeability into the skin and spreadability and can use to preserve and improve the skin health.

Example 17

Jelly ointment

A jelly ointment was prepared by conventional method according to the following formula.
(Formula) (the numbers behind ":" means "%")
Glycerol: 44.49
Ethanol: 30
Indigo-plant extract prepared by the method of Example 5: 15
Brine solution (hardness 54,000mg/ml): 5
Gellant (HIVIS Wako 104): 1.5
Polyoxyethylene (20) sorbitanmonooleate: 1.5
Photsensitizer 201: 0.01
2,2',2"-Nitriroethanol: 2.5
The product can be used as an agent for external application to the skin for skin-whitening, skin-beautifying and/or antiaging. This product also has excellent permeability into the skin and spreadability and can use to preserve and improve the skin health.

Example 18

Bath agent

A bath agent was prepared by conventional method according to the following formula.
(Formula) (the numbers behind ":" means ''%")
Hydrous crystalline $\alpha,\alpha$-trehalose (cosmetic grade, commercialized by Hayashibara Biochemical Laboratories, Inc.): 74.4
Sodium hydrogen carbonate: 12.5
Indigo-plant extract prepared by the method of Example 2: 7.5
Powdery product containing $\alpha, \alpha$-trehalose and borin in mass ratio of 144 : 202 prepared by the method described in Example 9 of International patent Application WO 2003/016325: 5.5
Photosensitizer 201: 0.0015
Perfume: adequate amount
Colorant: adequate amount
Filled up to make the total amount to 100%.
The product is a bath agent having excellent effect in skin-whitening and/or skin-beautifying.

Example 19

Mouth freshening film

A mouth freshening film was prepared by conventional method according to the following formula.
(Formula) (the numbers behind ":" means "%")
Pullulan (commercialized by Hayashibara Shoji, Inc.): 22
Carrageenan: 1
Xanthan gum: 0.15
Locust bean gum: 0.15
Maltitol: 0.8
Deionized water: 69.25
Indigo-plant extract prepared by the method of Example 1 (not containing 1,3-BG): 3
Mint oil emulsion: 2.6
Propolis extract: 0.5
Sucralose: 0.3
Citric acid: 0.25
Since the product contains indigo-plant extract, it has effect in keeping firmness and elasticity of gum-ridge and anti-inflammation and can be used for preserving and improving oral health. This product is also effective in breath care.

Example 20

Chewing gum

A chewing gum was prepared by conventional method according to the following formula.
(Formula) (the numbers behind ":" means "%")
Gum base: 30
Sucrose: 39.7
Indigo-plant extract prepared by the method of Example 4: 30
Perfume: adequate amount
Colorant: adequate amount
Filled up to make the total amount to 100%.
Since the product contains indigo-plant extract, it has effect in keeping firmness and elasticity of gum-ridge and anti-inflammation and can be used for preserving and improving oral health.

**INDUSTRIAL APPLICABILITY**

As described above, the indigo-plant extract forms little sediment, nasty smell and browning, and has high storage stability. The indigo-plant extract of the present invention has various physiological activities such as suppressing melanin production, inhibiting elastase activity, inhibiting lipase activity, regulating sebum secretion from glandula sebacea, SOD mimicking activity and scavenging DPPH radical, and the agent for external application to the skin containing the extract can be used for skin-antiaging and keeping youthful skin by restoring or preserving elastisity and firmness of skin by inhibition of elastase activity, as well as reducing color of pigmentation, fleck, freckle and chloasma caused by sunburn and skin-whitening. Since the above effects of the agent for external application to the skin of the present invention can be enhanced when used together with other skin-whitening ingredients, the agent for external application to the skin of the present invention is also usable for suppressing oxidation of sebum and skin-disorder caused by oxidation, antiaging of skin, protection of skin, therapy of athlete's foot, therapy of stomatitis, therapy of acne, prevention and tehraly of periodontal disease and caries. The agent for external application to the skin of the present invention can be comfortably used for a long term with acceptable level of side effect and safety and with good sense of use. The present invention, having these outstanding effects, is a significant invention that greatly contributes to this art.

**Claims**

1. An agent for external application to the skin comprising extract of indigo plant (*Polugonum tinctorium* Lour) as an effective ingredient, which is used for suppressing melanin production, inhibiting elastase activity and antiaging.

2. The agent of claim 1, wherein said extract is extracted with water or an aqueous solvent.

3. The agent of claim 1 or 2, which further comprises one or more substances selected from the group consisting of surfactant, preservative (antiseptic), humectant, thickener, water-soluble polymer, antioxidant, chelating agent, pigment, perfume, pH regulator, vitamins and additives.

4. The agent of any one of claims 1 to 3, which further comprises one or more substances selected from the group consisting of L-ascorbic acid derivative and salts thereof, alkoxysalicylic acid and salts thereof, hydroquinone glycoside and derivatives thereof, tranexamic acid and derivatives thereof, resorcinol derivative, koji acid and derivatives thereof, ellagic acid, linoleic acid, camomile extract and tetrahydrocurcuminoid.

5. The agent of claim 4, wherein said L-ascorbic acid derivative is selected from the group consisting of L-ascorbic acid alkylester, L-ascorbic acid phosphate ester and salts thereof, L-ascorbic acid sulfate ester and salts thereof and L-ascorbic acid 2-glucoside.

6. The agent of claim 4, wherein said alkoxysalicylic acid is selected from the group consisting of 4-methoxysalicylic acid and salts thereof.

7. The agent of claim 4, wherein said hydroquinone glycoside and derivatives thereof are selected from the group consisting of hydroquinone-$\alpha$-D-glucoside and hydroquinone-$\beta$-D-glucoside,

8. The agent of claim 4, wherein said resorcinol derivative is selected from the group consisting of alkylresorcinol and salts thereof and alkylresorcinol glycoside and salts thereof.

9. The agent of claim 4, wherein said tetrahydrocurcuminoid is selected from the group consisting of tetrahydrocucumin, tetrahydrodemethoxycurcumin and tetrahydro-bis-demethoxycurcumin.

10. The agent of any one of claims 1 to 9, which is used for skin-whitening, inhibiting lipase activity, regulating sebum secretion from glandula sebacea, eliminating active oxygen, scavenging radical, therapy of athlete 's foot, therapy of stomatitis, therapy of acne, therapy of eczema, therapy of eruption and therapy of periodontal disease.

Fig. 1

Fig. 2

Fig. 3

Fig. 4

| **INTERNATIONAL SEARCH REPORT** | International application No. |
|---|---|
| | PCT/JP2006/310555 |

A.  CLASSIFICATION OF SUBJECT MATTER
*A61K8/97*(2006.01), *A61Q19/02*(2006.01), *A61Q19/08*(2006.01), *A61K8/67*
(2006.01), *A61K8/60*(2006.01), *A61K36/70*(2006.01), *A61P1/02*(2006.01),
*A61P17/04*(2006.01), *A61P17/10*(2006.01), *A61P17/16*(2006.01),
According to International Patent Classification (IPC) or to both national classification and IPC

B.  FIELDS SEARCHED

Minimum documentation searched (classification system followed by classification symbols)
A61K8/60, A61K8/67, A61K8/97, A61K36/70, A61P1/02, A61P17/04, A61P17/10,
A61P17/16, A61P31/10, A61P39/06, A61P43/00, A61Q19/02, A61Q19/08

Documentation searched other than minimum documentation to the extent that such documents are included in the fields searched
Jitsuyo Shinan Koho          1922-1996    Jitsuyo Shinan Toroku Koho    1996-2006
Kokai Jitsuyo Shinan Koho    1971-2006    Toroku Jitsuyo Shinan Koho    1994-2006

Electronic data base consulted during the international search (name of data base and, where practicable, search terms used)
WPI

C.  DOCUMENTS CONSIDERED TO BE RELEVANT

| Category* | Citation of document, with indication, where appropriate, of the relevant passages | Relevant to claim No. |
|---|---|---|
| X<br>Y | JP 2000-344622 A  (Sunstar Inc.),<br>12 December, 2000 (12.12.00),<br>Claims; Par. Nos. [0020], [0029], [0030]<br>(Family: none) | 1-3,10<br>4-9 |
| A | JP 2001-069948 A  (Sunstar Inc.),<br>21 March, 2001 (21.03.01),<br>Claims; Par. No. [0022]<br>(Family: none) | 1-10 |
| X<br>Y | WO 2003/066433 A1  (Tetsuo SANTO),<br>23 October, 2003 (23.10.03),<br>Claims; page 4, line 48 to page 5, line 5<br>(Family: none) | 1-3,10<br>4-9 |

☒  Further documents are listed in the continuation of Box C.      ☐  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>23 June, 2006 (23.06.06) | Date of mailing of the international search report<br>11 July, 2006 (11.07.06) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

| International application No. |
|---|
| PCT/JP2006/310555 |

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2004-189732 A (Kabushiki Kaisha Tohoku Techno Arch),<br>08 July, 2004 (08.07.04),<br>Claims; Par. Nos. [0024], [0030] to [0034]<br>(Family: none) | 1,3,10<br>4-9 |
| Y | JP 2004-115381 A (Shiseido Co., Ltd.),<br>15 April, 2004 (15.04.04),<br>Claims; Par. No. [0001]<br>(Family: none) | 1-10 |
| P,X | JP 2005-179216 A (Kabushiki Kaisha Nomura),<br>07 July, 2005 (07.07.05),<br>Claims; Par. Nos. [0013], [0018], [0022]<br>(Family: none) | 1-4,10 |
| P,X | JP 2005-281253 A (NOF Corp., Kabushiki Kaisha Hayashibara Seibutsu Kagaku Kenkyusho),<br>13 October, 2005 (13.10.05),<br>Claims; Par. Nos. [0001], [0017], [0023]<br>(Family: none) | 1-4,10 |

Form PCT/ISA/210 (continuation of second sheet) (April 2005)

**INTERNATIONAL SEARCH REPORT**

International application No.

PCT/JP2006/310555

Continuation of A. CLASSIFICATION OF SUBJECT MATTER
   (International Patent Classification (IPC))

*A61P31/10*(2006.01), *A61P39/06*(2006.01), *A61P43/00*(2006.01)

   (According to International Patent Classification (IPC) or to both national
   classification and IPC)

Form PCT/ISA/210 (extra sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001031581 A
- JP 2004189732 A
- JP 6128133 A
- EP 1108419 A
- WO 0210361 A
- JP 2005095148 A
- JP 2005017642 W
- WO 2006035725 A1
- WO 2003016325 A

**Non-patent literature cited in the description**

- Saikin-Shinkin Kensa. Japan Public Health Association, 1982, 524-529